# EUROPEAN PATENT SPECIFICATION

(11) **EP 0 975 792 B1**
(45) Date of publication and mention of the grant of the patent: **10.10.2007**
(21) Application number: 98908909.9
(22) Date of filing: 04.03.1998
(51) Int. Cl.: C12Q 1/00, C07K 7/08, C07K 14/00, G01N 33/543, C07K 7/06, C07K 1/04

(54) **ISOLATION OF TISSUE SPECIFIC PEPTIDE LIGANDS AND THEIR USE FOR TARGETING PHARMACEUTICALS TO ORGANS**
ISOLIERUNG VON GEWEBSPEZIFISCHEN PEPTIDLIGANDEN UND IHRE VERWENDUNG ZUR AUSRICHTUNG VON PHARMAZEUTIKA AUF ZIELORGANE
ISOLEMENT DE LIGANDS PEPTIDIQUES A SPECIFICITE TISSULAIRE ET UTILISATION DE CES DERNIERS POUR CIBLER DES SUBSTANCES PHARMACEUTIQUES SUR DES ORGANES

(30) Priority: 04.03.1997 US 810074; 04.03.1997 US 39509 P
(43) Date of publication of application: 02.02.2000
(73) Proprietor: Savient Pharmaceuticals, Inc., East Brunswick New Jersey 08816 (US)
(72) Inventor: PANET, Amos, Jerusalem (IL); HAGAI, Yocheved, Rehovot (IL); LAZAROVITS, Janette, Re'ut (IL); NIMROD, Abraham, Rehovot (IL); VOGEL, Tikva, Rehovot (IL); LEVANON, Avigdor, Rehovot (IL); ZEELON, Elisha, Mishmar HaShiva (IL); BELKIND, Anna, 26217 Rehovot (IL); GOLAN, Itshak, Ashdod (IL)
(74) Representative: Vossius & Partner
(86) International application number: PCT/US1998/004188
(87) International publication number: WO 1998/039469

(56) References cited:
- WO-A-94/26787
- WO-A-97/10507
- US-A- 5 510 240
- PASQUALINI R ET AL: "ORGAN TARGETING IN VIVO USING PHAGE DISPLAY PEPTIDE LIBRARIES" NATURE, MACMILLAN JOURNALS LTD. LONDON, GB, vol. 380, 28 March 1996 (1996-03-28), pages 364-366, XP000986213 ISSN: 0028-0836

## Description

### BACKGROUND OF THE INVENTION

There is a need in the pharmaceutical industry for pharmaceutical agents that can be targeted to specific organs, and thus provide local drug delivery.

Advantages of local drug delivery are the lowering of the amount of drug needed to achieve therapeutic efficacy and the minimizing of undesired side effects.

Currently, the main approach to tissue specific targeting is either to infuse the drug through a catheter or a balloon (PTCA) to a site of the vasculature, or through linking of a drug to a protein ligand with affinity for a predetermined target.

For example, full-size monoclonal antibodies against predetermined cell surface antigens have been generated in order to produce cell targeting ligands. However, the complexity of isolating a specific antibody and the size of such antibody are severe limitations to their use as cell targeting ligands.

The use of a phage peptide display library facilitates an alternative means of producing unique ligands for targeting to specific, yet unrecognized cell (undetermined) surface moieties. Phage libraries have been used to select random peptides that bind to isolated pre-determined target proteins such as antibodies, hormone receptors, and the erythropoietin receptor.

Unlike the use of a known ligand, the process of phage selection from a random peptide library does not require prior knowledge of the target cell or its receptors. This approach also has the advantage that molecular recognition and ligand selection are not dependent on the immunogenicity of the candidate target protein, as required in the monoclonal antibody approach.

Pasqualini and Ruoslahti (Nature 380: 364-366, 1996) injected phage libraries intravenously into mice and subsequently rescued the phage from individual organs. Peptides capable of mediating selective localization of phage to brain and kidney blood vessels were identified and were shown to exhibit up to 13-fold higher sensitivity for these mouse organs. One of the peptides displayed by the brain-localized phage was chemically synthesized and shown to specifically inhibit the localization of the homologous phage into the brain. When coated onto glutaraldehyde fixed red blood cells, the peptide caused selective localization of intravenously injected red blood cells into the brain of the mouse.

The specification discloses the use of phage display peptide (epitope) libraries to identify peptides useful as ligands for targeting drugs, cells or genes to specific human tissue and various human organs, where the specific receptor is not predetermined. In addition, some experiments were carried out using predetermined targets.

A phage library is included in the organ perfusion fluid, and after *ex vivo* organ perfusion, phages are extracted from the human tissue, amplified and the displayed peptide sequence is determined. This *ex vivo* approach is applied to human organs such as placenta, umbilical cord artery and vein as well as blood vessels removed during surgery. This approach is further applied to diseased tissue removed during surgery and to organs such as kidney, heart and liver available following transplantation procedures.

The endothelium which lines the inner surface of blood vessels expresses multiple surface proteins and receptors for diverse types of ligands. Endothelial cells, derived from different tissues or even from veins and arteries of the same tissue, have been shown to be phenotypically arid functionally distinct. The unique distinctive, characteristic surface proteins and receptors expressed by endothelial cells of the various tissues are exploited to discover novel, defined peptide ligands which are subsequently linked to drugs or radioactive isotopes for targeting to the desired tissue.

The Specification provides a parallel approach to obtain unique, yet undetermined targets characteristic of lymphatic cells derived from different diseases, such as leukemia and autoimmune diseases. Since lymphocytes are obtained in suspension, biopanning is carried out by mixing a phage library with the lymphocyte cell suspension, followed by washing with several buffer solutions. Biopanning is repeated several times following amplification of selected phage population.

The peptide sequence of the subject invention, specific for different human organs and tissue cells are linked to various pharmaceutical agents to form drug-peptide conjugates and to radioactive isotopes for diagnostic and therapeutic purposes.

### Summary of the Invention

The subject invention provides a non-naturally occurring pharmaceutically active peptide for targeting a pharmaceutical agent or imaging agent to a tissue or organ comprising the amino acid sequence Met Arg Ala Pro Val Ile and the use of this peptide in the treatment of various diseases and conditions. The novel peptide specifically binds to undetermined targets (and some determined targets) in various organs and in lymphocytes.

The subject invention provides for compositions comprising the novel peptide and a pharmaceutical agent linked to the peptide, wherein the pharmaceutical agent is a polypeptide and is linked to the peptide by a peptide linkage. The pharmaceutical agent may also be a toxin, an anti-cancer agent, an anti-angiogenic compound, a cardiovascular agent, an agent used in a neurological disorder, a liver disease agent, a kidney disease agent or a radioisotope.

The subject invention provides for a method for the identification of the above peptide comprising incubating a phage display peptide library with an isolated organ; washing the isolated organ to remove unbound phages; eluting bound phage from the isolated organ; amplifying the resulting bound phage; and determining the displayed peptide sequence of the bound phage so as to identify the peptide.

The organ may be an artery, a vein, placenta, tumor tissue, kidney, heart, liver, or central nervous system. The organ may also be a perfused organ.

The phage display library may be a 15-mer library or a 6-mer library.

The elution medium may be a compound selected from acid, urea, Octyl, trypsin or tween.

The subject invention also provides for a method of producing the novel peptide comprising identifying the peptide as described above; and synthesizing the peptide by joining the amino acids of the peptide in the proper order.

The subject invention additionally provides an imaging agent which comprises the peptide of the subject invention with an imageable marker. Such an imaging agent may be used for diagnostic purposes.

The subject invention further provides a composition comprising an effective imaging amount of an imaging agent of the invention and a physiologically acceptable carrier.

The subject invention also encompasses a composition comprising an effective imaging amount of an imaging agent of the invention, a pharmaceutical agent linked thereto and a physiologically acceptable carrier.

The marker may be a radioactive isotope, an element which is opaque to X-rays or a paramagnetic ion.

The radioactive isotope may be indium-111, technetium-99, iodine-123, iodine-125, iodine-131, krypton-81m, xenon-33 or gallium-67.

The subject invention also provides for an ex vivo method for imaging an organ comprising contacting the organ to be imaged with an above defined imaging agent under conditions such that the imaging agent binds to the organ; imaging bound imaging agent; and thereby imaging the organ.

The subject invention also provides for the use of the peptide of the invention for the production of a pharmaceutical composition for treating an organ.

### Brief Description of the Figure

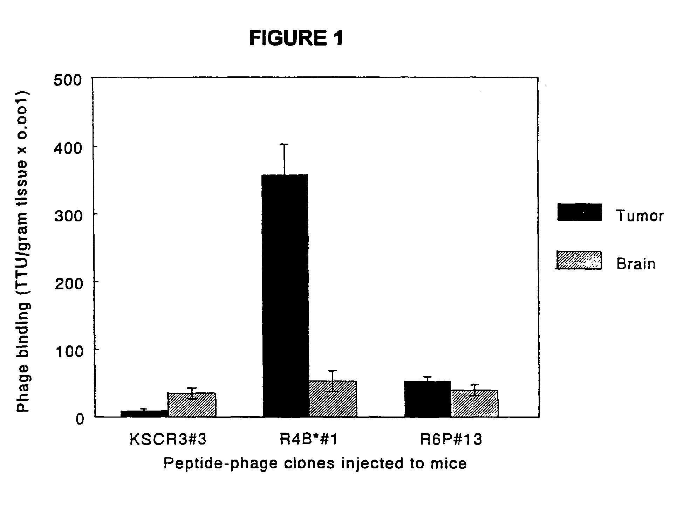

Figure 1: Organ distribution of selected phages
   Clone KSC#3 (KSCR3#3) is a phage clone that was enriched on Kaposi Sarcoma cells after three rounds of biopanning in culture. Clone R4B*#1 (TUV-R4B*#1) is a phage clone that was enriched on umbilical vein and artery. Clone #P13 (R6P#13) is a sporadic non-enriched phage clone as a negative control. The *in vivo* binding of these three clones to tumor tissue and brain tissue was compared.

### Detailed Description of the Invention

Phage display is a technique in which a peptide, antibody or protein is expressed on the surface of a bacteriophage, while the DNA encoding the displayed protein resides within the phage virion. A phage display peptide library (also termed phage peptide library or phage display library or phage library or peptide library) is constructed wherein the virions display a wide range of protein residues of specific lengths. This technology, known to one skilled in the art, is more specifically described in the following publications: Smith (1985) Science 228: 1315, Scott et al. (1990), Science 249: 386-390, Cwirla et al. (1990), P.N.A.S. 87: 6378-6382; Devlin et al. (1990), Science 249: 404-406, U.S. Patent Nos 5,427,908, 5,432,018, 5,223,409 and 5,403,484.

Biopanning is a procedure comprising many steps, one of which is selection; biopanning is carried out by incubating phages displaying protein ligand variants (a phage display library) with a target, washing away unbound phage and specifically eluting the bound phage. The eluted phage is amplified and taken through additional cycles of binding and amplification which enrich the pool of eluted specific sequences in favor of the best binding peptide bearing phages. After several rounds, individual phages are characterized, and the sequence of the peptides displayed is determined by sequencing of the corresponding DNA of the phage virion. A peptide obtained in this manner may be called a "lead-compound".

One way of obtaining a peptide with a higher affinity relative to a lead-compound is to construct an extension phage display peptide library based on a core amino acid sequence of the lead-compound. In such an extension library, random amino acids are added to each side of the core sequence.

An additional way to obtain a peptide with a higher affinity relative to a lead-compound is the construction of a phagemid display mutagenesis library. In such a library, oligonucleotides are synthesized so that each amino acid of the core sequence is independently substituted by any other amino acid.

The subject invention provides a polypeptide which comprises a peptide of the subject invention which corresponds to a peptide displayed on a phage virion and wherein both the polypeptide and the peptide have the same biological activity.

A peptide described described has less than 50 amino acids but more than 5 amino acids, e.g. 6-15 amino acids.

The peptide of the subject invention comprises 6 amino acids.

Several amino acids may be added either to the C-terminus or the N-terminus or to both termini of the peptide while maintaining the biological activity of the peptide, e.g 1-5 or 20-30 amino acids to each terminus. Further described is a Fv fragment of a human antibody of about 100 amino acids, displayed on the N-terminus of pIII of M13 bacteriophage. The peptide of the subject invention may have about 100 amino acids.

Cancer tissue as used herein may be obtained from any form of cancer such as carcinoma, sarcoma, leukemia, adenoma, lymphoma, myeloma, blastoma, seminoma or melanoma.

Diseased tissue as used herein may be obtained from any diseased organ such as liver, kidney, lung, heart, ovary, colon and so forth. The organ may be diseased as a result of an autoimmune disorder. The organ may be diseased as a result of any other disease, such as cardiovascular disease or cancer.

A neurologic disorder as used herein encompasses any neurologic disorder as defined and described in "The Merck Manual", sixteenth edition (1992). For example, muscular dystrophy, myasthenia gravis, multiple sclerosis, Alzheimer's disease, neuropathy, Parkinson's disease and amyotrophic lateral sclerosis (Lou Gehrig's disease) are neurologic disorders.

A vein as used herein may originate from any tissue. An example of a vein is safenal vein or femoral vein.

An artery as used herein may originate from any tissue, e.g. radial artery, coronary artery, mammary artery and so forth.

The peptide of the subject invention may be administered to a patient, alone, radiolabeled, linked to a pharmaceutical agent (drug), or in the form of a peptidomimetic.

The mode of administration of the peptide of the subject invention may be intravenous, intramuscular, subcutaneous, topical, intratracheal, intrathecal, intraperitoneal, rectal, vaginal or intrapleural.

The pharmaceutical agent may *inter alia* be a radioactive label (radio-isotope).

If the peptide or the peptide-drug combination is administered orally, it is administered in the form of a tablet, a pill or a capsule.

The compositions comprising the peptide produced in accordance with the invention may comprise conventional pharmaceutically acceptable diluents or carriers. Tablets, pills and capsules may include conventional excipients such as lactose, starch and magnesium stearate. Suppositories may include excipients such as waxes and glycerol. Injectable solutions comprise sterile pyrogen-free media such as saline and may include buffering agents, stabilizing agents or preservatives. Conventional enteric coatings may also be used.

Compositions for topical administration may be in the form of creams, ointments, lotions, solutions or gels.

The mode of administration of the peptide or drug-peptide linkage is a solid dosage form, a liquid dosage form, or a sustained-release formulation.

The subject invention provides the peptide comprising amino acids having the sequence: Met Arg Ala Pro Val Ile.

Further described are the following sequences: 
Gly Arg Gln His Phe Phe Leu Ala Glu Gly Arg Ser Phe Tyr Phe;

Ser His Val Pro Pro Ile Phe Asn Asp Val Tyr Trp Ile Ala Phe;

Val Pro Pro Ile Phe Asn Asp Val Tyr Trp Ile Ala Phe;

His Thr Phe Phe Leu Pro Gly Cys Ala Gly His Cys Ile Asp Ala;
 
Pro Ser Thr Thr Arg Asn Arg Thr Asp Ile Asn Lys Pro Thr Gln;

Phe Tyr Ser His Ser Ala Asp Gly Ala Arg Pro Phe Pro Leu Tyr;

Phe Tyr Ser His Ser Ala Asp Gly Ala Glu Ser Ser Pro Arg Met;

Phe Tyr Ser His Ser Ala Asp Gly Ala Pro Arg Arg Asp Leu Leu;

Phe Tyr Ser His Ser Ala Asp Gly Ala;

Phe Tyr Ser His Ser Ala Asp Gly Ala XXX XXX XXX Pro XXX XXX,
 wherein XXX is any amino acid; Phe Tyr Ser His Ser Ala Asp Gly Ala XXX XXX XXX XXX Leu XXX, wherein XXX is any amino acid; 
Gly Cys Gly Gly Phe His Pro Gly Ser Asn Cys Leu Val Val Ser;

Arg Asn Val Pro Pro Ile Phe Asn Asp Val Tyr Trp Ile Ala Phe;

Phe Leu Pro Asn Gly Phe;

Thr His Asp Thr His Leu;

Thr His Glu Thr Gln Arg;

Asp Ile Ala Lys Arg Tyr;

Asp Val Ser Pro Val Trp Ala Ala Phe Ala Ser Gly Ala Ser Phe;

Ser Asp Cys Leu His Ser Val Arg Gly Phe Asn Cys Leu Lys Arg;

Ser Leu Cys Phe Tyr Leu Phe Val Met Ser Ala Pro Asp Ala Pro;

Gly Leu Gly Gly Leu Ser Phe Gly His Ser Asp Asn Pro Pro Ser;

Gly Pro Gly Trp Val Gly Trp Phe Val Ser Leu Phe Tyr Ala Ser;
 
Trp Ser Leu Gly Ser Ser Trp Val Tyr Lys Phe Phe Tyr Ser Ser;

Ser Leu Trp Gly Ala Ser Ser Cys Gly Val Ala Phe Phe Glu Ser;
Gly Pos Pos Pos/Ar Ar Ar Leu Ala Glu Gly Arg Ser Ar Ar Ar
 wherein Pos is a positively charged amino acid and Ar is an aromatic amino acid.

Arg Ser Lys Tyr Arg Pro Asn Met Thr Asn

Leu Asn Pro Lys Val Lys His Met

Leu Arg Gly Gly Asn Ala Met

Val Ser Asp Arg Arg Gln Asn Val

Ser Lys Ser Pro

Gly Thr Leu Asn Gln Cys Gly Arg Ile Asn

Cys Ala Val Glu Ala Ala Gly Pro Val Arg Val Leu

Ser Gly Ser Leu Gly Arg Ser Leu Glu

Thr Gly Asp Glu

Phe Lys Ala Ser Arg His Ser

Ile His Met Arg Ala

Lys Asn Ala Asn

Gly Ile Lys Gly Leu Asp Glu

Cys Lys Trp Glu Lys Arg

Ala Arg Leu Ser Pro Thr Met Val His Pro Asn Gly Ala Gln Pro

Ala Leu Gly Gly Phe Arg Pro Phe Trp Ser Tyr Gly Gly Leu Ser

Met Gly Ala Asp Asp Ala Pro His Tyr Trp His Pro Val Trp Thr

Cys Thr Arg Leu Gly Ala Ala Ala Gly Arg Cys Asp Val Gly Leu

Arg Leu Phe Met Leu Gly

Pro Ile Trp His Gly Asp Ser Gly Val Tyr Ser Ser Phe Phe Pro

Arg Asn Thr Leu Pro His Phe Ser Phe Gly Pro Arg  Leu Tyr Arg

Gly Leu Ser Asp Gly pro Tyr Tyr Ser Phe Ser Leu Phe Arg Phe

Gly Gly Ala Ala Gly Gly Tyr Leu Arg Val Phe Ala Gly Val Arg

Gly Tyr Asp Cys Trp Asp Cys Pro Phe Ser Phe Arg Gly Ser Val

Ser Asn Leu Asn Arg Phe Val Phe Ala Phe Trp Asp Gly Pro Ala

Leu His Gly Phe Ala Ser His Lys Asp Gly Pro Leu Ile Pro Ala

Leu Val Phe Val Lys Asn His Pro Leu Val Pro Phe Gly Ser Pro

Ser Lys Arg Ala Asn Gly Phe Arg Gly Val Ser

Ile Lys His Tyr Gly Arg Lys Arg Asn

Val Lys Lys Phe Lys Gly Gly Gln Arg Val

In each of the sequences in this application the amino acid at the extreme left represents an amino acid on the amino terminal side of the peptide and the amino acid at the extreme right represents an amino acid on the carboxy terminal side of the peptide.

The invention further provides a composition comprising the peptide of the subject invention and a pharmaceutical agent linked thereto. The composition optionally also comprises a pharmaceutically acceptable carrier.

In a preferred embodiment, the pharmaceutical agent is a polypeptide and is linked to the peptide by a peptide linkage.

In another embodiment, the pharmaceutical agent is a toxin, an anti-cancer agent, an anti-angiogenic compound, a cardiovascular agent, an agent used in a neurological disorder, a liver disease agent or a kidney disease agent or a radio isotope.

In a presently preferred embodiment, the pharmaceutical agent is a recombinant protein.

The subject invention further provides a composition comprising a peptide of the subject invention and a pharmaceutically acceptable carrier.

Also described is a chimeric polypeptide comprising a first peptide and a second peptide wherein the first peptide is the peptide of the subject invention.

The second peptide may be a toxin, an anti-cancer agent, an anti-angiogenic compound, a cardiovascular agent, an agent used in a neurological disorder, a liver disease agent or a kidney disease agent, or a recombinant protein.

The subject invention also provides a method for the identification of a peptide which comprises incubating a phage display peptide library with cells of an isolated organ, washing the isolated organ to remove unbound phages, eluting bound phage from the isolated organ, amplifying the resulting bound phage and determining the displayed peptide sequence of the bound phage so as to identify the peptide.

The subject invention also encompasses a method of synthesizing the peptide of the subject invention which comprises joining the amino acids of the peptide in the proper order.

The subject invention further provides a method of producing a peptide which comprises identifying the peptide by the above described method and synthesizing the peptide by joining the amino acids of the peptide in the proper order.

In a preferred embodiment, the isolated organ is a perfused organ.

In another embodiment, the isolated organ is an artery, a vein, placenta, tumor tissue, kidney, heart, liver or central nervous system.

In yet another embodiment, the artery is umbilical cord artery, a radial artery, a coronary artery, a mammary artery or a damaged artery.

In an especially preferred embodiment, the damaged artery is a damaged coronary artery.

In a preferred embodiment, the vein is umbilical cord vein, safenal vein or femoral vein.

It is envisaged that the phage display peptide library is a 15-mer library, a 6-mer library or a synthetic human antibody library.

Preferred elution medium is a compound selected from acid, urea, Octyl, trypsin or tween.

The subject invention additionally provides an imaging agent which comprises the peptide of the subject invention with an imageable marker. Such an imaging agent may be used for diagnostic purposes.

The subject invention further provides a composition comprising an effective imaging amount of an imaging agent of the invention and a physiologically acceptable carrier.

The subject invention also encompasses a composition comprising an effective imaging amount of an imaging agent of the invention, a pharmaceutical agent linked thereto and a physiologically acceptable carrier.

In a preferred embodiment, the marker is a radioactive isotope, an element which is opaque to X-rays or a paramagnetic ion.

In a presently preferred embodiment, the radioactive isotope is indium-111, technetium-99, iodine-123, iodine-125, iodine-131, krypton-81m, xenon-33 or gallium-67.

The subject invention also provides an ex vivo method for imaging an organ which comprises contacting the organ to be imaged with an imaging agent of the invention under conditions such that the imaging agent binds to the organ, imaging the bound imaging agent and thereby imaging the organ.

In a preferred embodiment, the organ is an artery, a vein, placenta, tumor tissue, kidney, heart or liver.

In another embodiment, the artery is umbilical cord artery, a radial artery, a coronary artery, a mammary artery or a damaged artery.

In another embodiment, the damaged artery is a damaged coronary artery.

In another embodiment, the vein is umbilical cord vein, safenal vein or femoral vein.

In another embodiment, the pharmaceutical agent is a polypeptide and is linked to the imaging agent by a peptide linkage.

In a preferred embodiment, the pharmaceutical agent is a toxin, an anti-cancer agent, an anti-angiogenic compound, a cardiovascular agent, an agent used in a neurological disorder, a liver disease agent or a kidney disease agent.

The subject invention further provides a composition wherein the pharmaceutical agent is a recombinant protein.

The subject invention further provides the use of the composition of the invention for the preparation of a pharmaceutical composition for treating an organ.

In a preferred embodiment, the organ is an artery, a vein, placenta, tumor tissue, kidney, heart, liver, or central nervous system.

In another embodiment, the artery is umbilical cord artery, a radial artery, a coronary artery or a mammary artery or a damaged artery.

In another embodiment, the damaged artery is a damaged coronary artery.

In another embodiment, the vein is umbilical cord vein, safenal vein or femoral vein.

The novel peptide of the subject invention or its corresponding peptidomimetics may also be used in the manufacture of compositions to treat various diseases and conditions.

The specification describes a method for the identification of peptides or antibodies by biopanning which comprises incubating a phage display library with lymphocytes derived from blood, washing to remove unbound phages, eluting the bound phages from the lymphocytes, amplifying the resulting bound phage and determining the displayed peptide sequence of the bound phage so as to identify the peptide.

### EXAMPLE 1: Isolation and Selection of Tissue-Specific Epitopes which Specifically Bind to Undetermined Targets in Umbilical Cord Vein and Artery

### 1. MATERIALS AND METHODS

### 1.1. Preparation of perfused umbilical cord vein and artery

Freshly obtained full term umbilical cord was cut into fragments of 4-5 cm in length, the blood was removed and the umbilical cord was cannulated and connected either through the vein (V) or the artery (A) to a small circulating pump for perfusion at a rate of 1ml/min (Pharmacia peristaltic pump). Perfusion temperature was either 4°C or 23°C and the perfusion buffer composition is indicated below.

### 1.2. Peptide display phage library source

Two phage display peptide libraries (6-mer and 15-mer) were kindly provided by G. Smith (Virology 167: 156-165, 1988). The libraries were amplified to form phage library working stock.

The libraries were originally constructed by splicing the 2.8 kilo-base pair tetracycline resistance determinant of Tn10 into the minus-strand origin of replication of the wild type fd phage. The resulting defect in minus strand synthesis reduced the intracellular replicative form (RF) copy number to 1. As a result thereof, the filamentous phage including fd-tet do not kill their host. The infected cell, which becomes resistant to tetracycline, continues to grow and secret tetracycline resistant progeny particles (about 10 tetracycline transducing units (TTU) per bacteria). The infectivity of the 6-mer and 15-mer libraries are 2.5% and 39%, respectively. The calculated theoretical number of primary clones for the 6 and 15-mers libraries are 4x10⁷ and 1x10¹⁹, respectively. However, the number of primary clones obtained from each library was about 2x10⁸. The phage titer is determined by infecting starved K91/kan bacterial culture and selecting tetracycline resistant clones on kan/tetracycline agar plates. The biopanning yield is calculated as the % of the output phage from the total input.

### 1.3. Bacterial strain: K91/Kan

The preparation of starved bacterial culture for phage infection was essentially as described by G. Smith (1993), Methods of Enzymology 217:228.

### 1.4. Phage selection and amplification

Phages that expressed epitopes of specific interest were selected from the libraries by biopanning in an essentially 4-step procedure:
a. Binding of phage to the perfused vessel cells
b. Removal of non-bound phage by extensive washing
c. Elution of bound phages
d. Infection and propagation of eluted phage in *E. coli.*

This biopanning procedure was generally repeated 4-6 times. Selected clones were individually propagated, and the single stranded DNA from the secreted phage was purified. Properties of the selected clones were examined as follows:
(i) Sequencing selected phage:
   The DNA sequence of the insert was determined by the dideoxy DNA sequencing method (Sanger et al. (1977), P.N.A.S. 74: 5463-5467) using Sequenase Version 2.0 (DNA Sequencing Kit, Amersham) and a primer of 18 nucleotides (5': TGAATTTTCTGTATGAGG).
(ii) Biopanning and tissue distribution *in vivo* (mouse or rat)
(iii) Phage binding comparison *ex vivo,* in culture, and *in vivo*

The following procedures are then carried out (Example 4):
(iv) Peptide synthesis
(v) Mutant peptide synthesis
(vi) Peptide cross linking (chemically and by genetic engineering)
(vii) Immunolocalization (using labeled antibody)
(viii) Linking of peptide to drug
(ix) Radiolabeling of peptide
(x) Radiotherapy and other therapeutic treatments.
(xi) Organic synthesis of peptidomimetic.

### 1.5 Basic Biopanning protocols

Several biopanning protocols were developed and used:
1.5.1. Protocol T1 (Acid/Urea/Octyl elution)
1.5.2. Protocol R1 (Trypsin-EDTA/Acid-Tween elution)
1.5.3. Protocol N1 (Acid-Tween/Tissue elution)
1.5.4. Protocol R2 (Trypsin-EDTA/Acid-Tween/Tissue elution)
1.5.5. Protocol EC-1 (Acid/Tissue/Urea elution)

### 1.5.1. Protocol T1 (Acid/Urea/Octyl elution)

1. Prewashing of the umbilical cord was carried out with 20 ml cold PBS¹ followed by 20ml cold DMEM-5% BSA.
   ¹ PBS is 136 mM NaCl, 2.7 mM KCl, 100 mM Na₂HPO₄. 2H₂O, 1.4 mM KH₂PO₄
2. Selection was carried out at 2x10¹⁰ TTU² in 3ml, DMEM³-1% BSA⁴-Protease inhibitors mixture ⁵(Pi). (50µl of the selection solution is used for titration of the input phage).
   ² TTU is phage infection units, conferring tetracycline resistance in the *E.coli* host.
   ³ DMEM is Dulbecco's Modified Eagle Medium with D-glucose (450 mg/l), Bet HaEmek
   ⁴ BSA is bovine serum albumin fraction V (Sigma)
   ⁵ Pi is Phenyl Methyl Sulfonyl Fluoride (PMSF) 1mM, Aprotinin (20µg/ml), Leupeptin (1 µg/ml)
3. Wash 1+ Wash 2 (W1,W2) was carried out in 2 x 25ml DMEM-5% BSA-Pi. When indicated, 0.2% Tween-20 was included.
4. Wash 3 (WO) was carried out in 3 ml W1+2 containing TBS⁶-Octyl⁷(0.05%).
   ⁶ TBS is 50 mM Tris-HCl, pH 7.5 and 150 mM NaCl
   ⁷ Octyl is octyl-β-D-glucopyranoside (Sigma)

Steps 2-4 were carried out at 4°C or at 23° C as indicated below.
5. Elutions: eluates were collected into 6ml TBS-1% BSA and adjusted to pH 7.4 with Trisma Base.
   5a. Acid elution was carried out in 3 ml 0.2M glycine-HCl, pH 2.2.
   5b. Urea elution was carried out in 3 ml 6M urea, pH3 (in 0.2M Glycine-HCl, pH3.0).
   5c. Octyl elution was carried out in 3ml TBS containing 0.2% Octyl.
6. Concentration/dialysis of the eluates was carried out on a 30K millipore dialysis membrane.
7. Amplification: for the first biopanning round, the entire eluate was amplified. For other rounds of biopanning only 20% of the eluate was amplified. The amplification was carried out in liquid medium by mixing the eluate with equal volume of 10¹⁰ starved bacteria. After 10 minutes, 100ml NZY⁸ solution containing 0.2 µg/ml tetracycline was added and bacterial suspension was mixed vigorously at 37°c for 30 minutes. Diluted samples were plated immediately on agar/kanamycin/tetracycline plates for titration of the output phage. To the rest of the bacterial cell suspension 100*µ*l of 20mg/ml tetracycline were added and incubation/amplification continued over-night.
   ⁸ NZY is a medium consisting of 10 g NZ amine A, 5 g yeast extract and 5 g NaCl.

### 1.5.2. Protocol R1 (Trypsin-EDTA/Acid-Tween elution)

1. Prewashing of umbilical cord vein and artery was carried out with 30ml DMEM-heparin (5u/ml) (Laboratoire Choay)-10⁸ M13 phage followed by 30ml, DMEM-5% human serum (Sigma).
2. Selection was carried out with 30ml, DMEM-5% human serum containing phage, 2×10¹⁰ TTU.
3. Wash was carried out with 40ml DMEM 5% human serum in the presence of Pi.
4. Elutions were carried out with:
   a. Trypsin-EDTA (2 ml, 0.25% and 0.05% respectively).
   b. Acid-Tween (5ml, 0.2M glycine-HCl (pH2.2)-0.5% Tween).
5. Titration and amplification:
   The acidic fraction was neutralized with 2M Trisma base (about 270µl). To the combined 7ml eluate, 200µl, 10¹⁰ bacteria and 10ml NZY were added. The solution was mixed gently for 10 minutes at 37°C. 100ml prewarmed NZY and 0.2*µ*g/ml tetracycline was added and mixed vigorously for 30 minutes. 100*µ*l 20mg/ml tetracycline was added and steps 1-4 were continued on ice and step 5 at room temperature.

### 1.5.3. Protocol N1 (Acid-Tween/Tissue elution)

1. Prewashing was carried out with 30ml DMEM-heparin (5u/ml) containing 10⁸ M13 phage followed by 30ml DMEM-5% human serum.
2. Selection was carried out with 30ml DMEM-5% human serum, containing 4x10¹⁰ TTU.
3. Wash was carried out with 40ml DMEM-5% human serum and 2% human haemoglobin (Sigma)-Pi.
4. Elution 1 was carried out in 3ml acid (0.2M glycine-HCl (pH2.2), 1% BSA, 0.5% Tween-20). The blood vessel was further washed with 2ml DMEM-5% human serum and combined with elution 1. 200µl 10¹⁰ bacteria and 10ml NZY were added, mixed gently and left at room temperature for 10 minutes.
   Elution 2, tissue elution (i.e. bacteria elution), was carried out by clamping one side of the vein and adding 0.5ml NAP⁹ buffer containing 2.5 x 10¹⁰ starved bacteria. After clamping the other end of the vein, the blood vessel was immersed into DMEM- 1% BSA solution and shaken at 37°C for 45 minutes. The bacteria were removed to a 50ml tube, the blood vessel was washed twice with 1-2ml NAP buffer, and the two washes were combined. 10ml NZY was added, mixed gently and left at room temperature for 10 minutes.
   ⁹ NAP consists of 80mM NaCl and 50 mM NH₄H₂PO₄, pH 7.0
   Steps 1-4 were carried out on ice and step 5 at room temperature (using a lamp from above).
5. Titration and amplification:
   100ml prewarmed NZY containing 0.2 *µ*g/ml tetracycline was added and mixed vigorously for 30 minutes. Samples (50 *µ*l each) were removed for titration on plates (output), and for over-night amplification, 100µl of 20mg/ml tetracycline was added.

### 1.5.4. Protocol R2 (Trypsin-EDTA/Acid-Tween/Tissue elution)

Protocol R2 is essentially identical to protocol R1 apart from the addition of tissue elution with bacteria following the trypsin and acid-tween elutions.

### 1.5.5. Protocol EC-1

Primary human endothelial cells seeded in 35cm tissue culture bottles (3rd passage of full term umbilical cord) were used.
1. Re-equilibration to 37°C and CO₂ for 30 minutes.
2. Prewashing was carried out with 5ml serum-free medium followed by 10 ml blocking solution (DMEM-BSA(1%)-Pi) for 90 minutes at 37°C.
3. Selection (incubation) for 45 minutes at 37°C with 3ml DMEM-BSA(1%)-Chloroquine (100*µ*M) mixture containing 2×10¹⁰ phages of the 15-mer initial library and a selected phage clone at a ratio of approximately 100:1, respectively.
4. Washing was carried out 5 times with 5ml blocking solution for 5 minutes each at room temperature.
5. Elutions:
   5a. Elution 1 was carried out with 2ml acid-glycine pH 2.2 containing 0.2% Tween for 10 minutes at room temperature. 170µl of 2M Trizma base was added to the bottle for neutralization, and the acid elution obtained was transferred to another tube. The bottle was washed with 2ml DMEM-BSA(1%). 0.2ml of the eluate was mixed with NAP buffer containing 1x10¹⁰ starved bacteria, and after 10 minutes absorption at room temperature, 2ml NZY-0.2% tetracycline was added and the suspension was shaken at 37°c. Aliquots were plated on agar-kanamycin-tetracycline plates for phage titration.
   5b. Elution 2 was carried out with 2ml NaPO₄ buffer containing 5x10¹⁰ starved bacteria. Incubation was carried out at room temperature for 45 minutes on a rocker. Cell eluate was transferred to another tube. The bottle was washed with 2ml medium. 0.4ml suspension was mixed with 2ml NZY 0.2%-tetracycline and incubated and titrated as described for elution 1(6a).
   5c. Elution 3 was carried out with 2ml urea (6M, pH 3). Incubation at room temperature for 10 minutes while rocking. 170µl of 2M Trizma base was added directly to the bottle and urea eluate was transferred to another tube containing 36ml medium. The bottle was washed with 2ml medium. 0.2ml was mixed with 0.2ml NAP buffer containing 1x10¹⁰ starved bacteria and then continued as described for elution 1 (6a)

### Testing the working stock of 15-mer library:

The working stock of the 15-mer library was the product of several cycles of amplifications. Several clones of the working stock (i.e. input of round 1) were isolated, amplified and their single stranded DNA was purified and sequenced. None of the phage clones was identical to specifically selected clones described below.

In addition, approximately 500 colonies derived from the same library were examined by colony hybridization to the ³²P-labeled antisense oligonucleotide probe of clones TUV-R4B-#1 and TUV-R4B-#3 disclosed below. None of the colonies hybridized to these probes.

### 2. RESULTS:

### 2.1. Specificity of phage library to umbilical cord

**Table 1: Comparison of phage binding to umbilical cord vein and non-specific phage adsorption to peristaltic pump plastic tubes following biopanning with 15-mer library (yield after acid and urea elution, protocol T1, room temperature (23°C)).**

| | Plastic tubes¹ | yield % | Vein² | yield % |
|---|---|---|---|---|
| | (TTU) | | (TTU) | |
| input | 2x10⁹ | | 3x10¹⁰ | |
| W1+ Tween | 1x10⁸ | 6 | 1x10⁹ | 3.8 |
| W2 + Tween | 2x10⁶ | 0.1 | 9x10⁶ | 0.03 |
| WO | 2x10⁵ | 0.001 | 1x10⁵ | 0.0004 |
| acid elution | <<10³ | <.00001 | 7x10⁶ | 0.02 |
| urea elution | <<10³ | <.00001 | 9x10⁶ | 0.0260 |

| | | | | |
|---|---|---|---|---|
| ¹ Selection was carried out through the circulating pump without the umbilical vein. ² Selection was carried out through the umbilical vein and the circulating pump. | | | | |

Thus, the results shown in Table 1 indicate that the phage yield values of the acid and the urea elutions from the umbilical vein were 2 to 3 orders of magnitude higher than the background values obtained from background binding to the plastic tubes, thus indicating a specific binding of the phage to the umbilical vein.

### 2.2 Effect of temperature and detergent on phage binding

**Table 2: Effect of temperature and Tween-20 on the binding of the 6-mer and 15-mer phage libraries to umbilical artery and vein (acid/urea/Octyl elution, protocol T1).**

| | **Artery (4°C), 6-mer-** | **Vein 1 (4°C), 15-mer-** | **Vein 2 (23°C),** |
|---|---|---|---|
| | **(TTU)** | **(TTU)** | **15-mer (TTU)** |
| Input | 2.6x10¹⁰ | 2.5x10¹⁰ | 1.8x10¹⁰ |
| W1 | 1.7x10¹⁰ | 2.5x10¹⁰ | |
| W2 | 1.4x10⁹ | 2.8x10⁹ | |
| W1+Tween | | | 1.3x10¹⁰ |
| W2+Tween | | | 9x10⁶ |
| WO | 2.8x10⁸ | 6.5x10⁸ | 1.3x10⁵ |
| Acid Elution | 3.1x10⁶ | 1.5x10⁶ | 6.6x10⁶ |
| Urea Elution | 3.0x10⁶ | 3x10⁶ | 9.0x10⁶ |
| Octyl Elution | 3x10⁸ | 2.4x10⁸ | 1.1x10⁶ |

The results indicate that by the addition of Tween (0.2%) in the washing solution and by increasing the temperature of the biopanning procedure to 23°C, there is a dramatic decrease in phage titers of the subsequent washing and elution steps (i.e. W2 + Tween, WO and Octyl elution) both with artery and vein. Therefore, the conditions of vein 2 (i.e. biopanning at 23°C and washing with a buffer containing 0.2% Tween followed by washing with a buffer containing 0.05% Octyl) were selected and used in the following protocol T1 based experiments.

### 2.3 Yield of Biopanning with Umbilical Vein and Artery

**Table 3: Biopanning of 15-mer library with human umbilical vein (Vein 1 and Vein 2) and 6-mer library with human umbilical artery (Artery 1) - yield after urea elution, protocol T1 (experiment B).**

| Round # | Artery 1 (4°C) | Vein 1 (4°C) | Vein 2 |
|---|---|---|---|
| | (TTU) | (TTU) | (23°C) |
| | | | (TTU) |
| ^{a}R1B | 1.7x0⁻² | 2x10⁻² | 2.3x10⁻² |
| ^{b}R2B | 0.9x10⁻² | 0.8x10⁻² | 2x10⁻² |
| ^{c}R3B | 1.2x10⁻¹ | 1x10⁻¹ | 1.3x10⁻¹ |
| ^{d}R4B | 1.5x10⁻¹ | 1.3x10⁻¹ | 1.28x10⁻¹ |
| ^{e}R4B* | - | - | 3x10⁰ |

| | | | |
|---|---|---|---|
| The protocol used was essentially as protocol T1 with indicated modifications below. Input phage in rounds R1B-R4B was 2x10¹⁰-10¹¹ and in R4B* was 1x10⁹. a. Selection was carried out for 15 minutes. Wash was carried out in buffer without Tween, followed by buffer containing Octyl. b. Selection was carried out for 8 minutes. Vein 2 was washed with buffer containing Tween, followed by buffer containing Octyl. Artery 1 and Vein 1 were washed with buffer without Tween followed by buffer containing Octyl. Elutions were carried out for 8 minutes. c. Selection was carried out for 3 minutes. Washing was carried out with buffer containing Tween followed by buffer containing Octyl for all blood vessels. Elution was carried out for 8 minutes. d. and e. Selection and washing conditions were identical to c. Elution was carried out for 3 minutes. | | | |

Twenty-eight clones were sequenced after R4B*V2, i.e. the urea eluted fraction from umbilical vein 2 after round 4:
17/28 (60%) of the clones were identical: this clone was designated TUV-R4B*-#1:
**Amino Acid Sequence displayed by clone TUV-R4B*-#1:**

Gly Arg Gln His Phe Phe Leu Ala Glu Gly Arg Ser Phe Tyr Phe
1 2 3 4 5 6 7 8 9 10 11 12 13 14 15

Six amino acids are aromatic (bolded) forming two hydrophobic clusters (amino acids 4,5,6,...13,14,15), accompanied by a linker peptide (underlined) region containing the charged sequence Glu Gly Arg (9,10,11) followed by Ser Phe. This sequence is similar to the NGRSF (Asn Gly Arg Ser Phe) sequence motif obtained by selection of phage bound to the α5β1 integrin (E. Koivunen et al., 1994, Methods in Enzymology 245: 346-367). In addition, the amino end of this peptide is positively charged having the sequence Arg, Gln, His (position 2, 3, 4, double underlining).
3/28 (11%) identical clones were designated TUV-R4B*-#3:
**Amino Acid Sequence displayed by clone TUV-R4B*-#3:**

Ser His Val Pro Pro Ile Phe Asn Asp Val Tyr Trp Ile Ala Phe
1 2 3 4 5 6 7 8 9 10 11 12 13 14 15

The underlined sequence also appears in clone TUV-R5D-#2 (see below).
2/28 (7%) were identical clones and were designated TUV-R4B*-#11.
**Amino Acid Sequence displayed by clone TUV-R4B*-#11:** 
His Thr Phe Phe Leu Pro Gly Cys Ala Gly His Cys Ile Asp Ala
1 2 3 4 5 6 7 8 9 10 11 12 13 14 15
2/28 (7%) were identical clones and were designated TUV-R4B*-#23.
**Amino Acid Sequence displayed by clone TUV-R4B*-#23:** 
Pro Ser Thr Thr Arg Asn Arg Thr Asp Ile Asn Lys Pro Thr Gln
1 2 3 4 5 6 7 8 9 10 11 12 13 14 15
4/28 were unique clones.

Furthermore, ten clones were sequenced after R4BV2, i.e. another urea eluted fraction from umbilical vein 2 after round 4:
3/10 (30%) clones were identical to clone TUV-R4B*-#1 and 7/10 were unique clones.

In addition, seven clones were sequenced after R4BA1, i.e. the urea eluted fraction from umbilical artery after round 4: all seven clones were unique clones.

**Table 4: Biopanning of 15-mer library with umbilical vein - yield of acid elution fraction using Protocol T1 (Experiment B)**

| Round# | Input (TTU) | Output (TTU) | Yield(%) |
|---|---|---|---|
| R3Ba¹ | 3x10¹⁰ | 7x10⁶ | 2x10⁻² |
| R4Ba | 7x10⁹ | 5x10⁵ | 6x10⁻³ |
| R5Ba | 4x10¹⁰ | 2x10⁶ | 6x10⁻³ |
| R6Ba | 3x10¹⁰ | 3x10⁶ | 9x10⁻³ |

| | | | |
|---|---|---|---|
| ¹ The amplified acid elution of R2B-V2 (Table 3) was used as the input of round 3. In the subsequent rounds (R4Ba-R6Ba) the amplified acid eluted phage was used for biopanning. | | | |

Ten clones were sequenced after R6Ba, i.e. the acid eluted fraction from umbilical vein 2 after round 6:
4/10 clones were identical and displayed strong homology to clone TUV-R4B*-#3. This clone was designated TUV-R6Ba-#7.
**Amino acid sequence displayed by TUV-R6Ba-#7:**

Arg Asn Val Pro Pro Ile Phe Asn Asp Val Tyr Trp Ile Ala Phe
1 2 3 4 5 6 7 8 9 10 11 12 13 14 15

The bolded amino acid sequence appears in clone TUV-R4B*-3# as well. Moreover, this clone is identical to LTV-R5D-#2 (below). Thus, TUV-R6Ba = TUV-R5D-#2.

In addition, 1/10 clone was identical to TUV-R4B*-#1.

**Table 5: Biopanning of 15-mer library with umbilical vein - yield of Octyl elution fraction using Protocol T1 (Experiment B)**

| Round # | Input (TTU) | Output (TTU) | Yield (%) |
|---|---|---|---|
| R3BO² | 3x10¹⁰ | 1x10⁶ | 3x10⁻³ |
| R4BO | 3x10¹⁰ | 5x10⁶ | 2x10⁻³ |
| R5BO | 2x10¹¹ | 7x10⁵ | 4x10⁻⁴ |
| R6BO | 1×10¹⁰ | 4x10⁵ | 4x10⁻³ |

| | | | |
|---|---|---|---|
| ² The amplified Octyl eluate of R2B-V2 (Table 3) was used as the input of round 3. In the subsequent rounds (R4BO-R6BO) the amplified Octyl eluted phage was used for biopanning. | | | |

Seven clones were sequenced after R4BO, i.e. the Octyl eluted fraction from umbilical vein 2 after round 4:
6/7 (85%) clones were identical to clone TUV-R4B*-#1.

Table 6: Biopanning of 15-mer library with umbilical vein - yield after acid-tween/trypsin elution, protocol R1 (experiment C) :

**I. Yields of Rounds 1-5 with umbilical vein**

| | Input (TTU) | Output (TTU) | Yield(%) |
|---|---|---|---|
| R1C-Vein | 5x10⁸ | 1.5x10⁶ | 0.03 |
| R2C-Vein | 3.3x10¹⁰ | 2.8x10⁶ | 0.0085 |
| R3C-Vein | 4.7x10¹⁰ | 6.7x10⁵ | 0.0014 |
| R4C-Vein | 2.4x10¹⁰ | 8.4x10⁵ | 0.0035 |
| R5C-Vein | 1.3x10¹⁰ | 4.9x10⁶ | 0.037 |

**II. Yields of Round 6 with umbilical vein and artery.**

| | Input (TTU) | Output (TTU) | Yield (%) |
|---|---|---|---|
| R6C-Vein | 1.5x10¹¹ | 6.4x10⁶ | 0.004 |
| R6C-Artery | 1.5x10¹¹ | 1.7x10⁶ | 0.001 |

Seven clones were sequenced after R4C, i.e. the Acid-Tween/Trypsin eluted fraction from umbilical vein after round 4:
6/7 (85%) clones were identical to clone TUV-R4B*-#1.

Furthermore, seven clones were sequenced after R5C, i.e. the Acid-Tween/Trypsin eluted fraction from umbilical vein after round 5:
7/7 clones were identical to clone TW-R4B*-#1.

The binding of clone TUV-R4B*-#1 to the umbilical vein (R6C-V) and to the artery (R6C-A) was nearly identical, indicating the inability of clone TUV-R4B*-#1 to discriminate between the two types of vessels.

**Table 7: Biopanning of umbilical vein with a mixture of the 6-mer and the 15-mer libraries (10¹⁰ TTU each)- yield of tissue elution with protocol N1 (Experiment D)**

| Round # | Input (TTU) | Output (TTU) | Yield (%) |
|---|---|---|---|
| R1D | 2x10¹⁰ | 2x10⁷ | 10⁻¹ |
| R2D | 5x10¹⁰ | 4x10⁷ | 8x10⁻² |
| R3D | 3x10¹¹ | 2x10⁶ | 10⁻³ |
| R4D | 2x10¹⁰ | 8x10⁶ | 4x10⁻² |
| R5D | 3x10₁₀ | 7x10⁶ | 3x10⁻² |

Six clones were sequenced after R4D, i.e. the tissue eluted fraction from umbilical vein after round 4:
3/6 clones (clones #1, #2, and #4) were identical at nucleotide positions 1-27 (amino acids 1-9). Clones #1 and #2 also have an identical amino acid at position 13 (proline) and clones #1 and #4 have an identical amino acid at position 14 (leucine).

These clones were designated TUV-R4D-#1, TUV-R4D-#2, and TUV-R4D-#4.
**Amino Acid Sequence displayed by clone TUV-R4D-#1** 
Phe Tyr Ser His Ser Ala Asp Gly Ala Arg Pro Phe Pro Leu Tyr
1 2 3 4 5 6 7 8 9 10 11 12 13 14 15
**Amino Acid Sequence displayed by clone TUV-R4D-#2:** 
Phe Tyr Ser His Ser Ala Asp Gly Ala Glu Ser Ser Pro Arg Met
1 2 3 4 5 6 7 8 9 10 11 12 13 14 15
**Amino Acid Sequence displayed by clone TUV-R4D-#4:**

Phe Tyr Ser His Ser Ala Asp Gly Ala Pro Arg Arg Asp Leu Leu
1 2 3 4 5 6 7 8 9 10 11 12 13 14 15

The amino acids bolded in clones TUV-R4D-#1, TW-R4D-#2 and TUV-R4D-#4 are the amino acids which the displayed peptide sequences have in common.
3/6 clones were unique (#3, #5, and #6) clones.
**Amino Acid Sequence displayed by clone TUV-R4D-#3:**

Gly Cys Gly Gly Phe His Pro Gly Ser Asn Cys Leu Val Val Ser
1 2 3 4 5 6 7 8 9 10 11 12 13 14 15

This peptide is identical to clone YCA-4R4-#10 in Example 3 below. Thus, TUV-R4D-#3 = YCA-4R4-#10.

TUV-R4D-#3 was also identical to six of the clones selected at R5D, to two of the clones selected at R5E and to seven clones selected at R5H.

In addition, ten clones were sequenced after R5D, i.e. the tissue eluted fraction from umbilical vein after round 5:
6/10 clones were identical to clone TUV-R4D-#3.
4/10 identical clones were designated TUV-R5D-#2.
**Amino Acid Sequence displayed by clone TUV-R5D-#2**

Arg Asn Val Pro Pro Ile Phe Asn Asp Val Tyr Trp Ile Ala Phe
1 2 3 4 5 6 7 8 9 10 11 12 13 14 15

The underlined sequence appears in clone TUV-R4B*-#3 as well. Furthermore, this clone is identical to clone YCA-5R1-#11 (Example 3). Thus, TUV-R5D-#2 = YCA-5R1-#11

**Table 8: Biopanning of umbilical artery with a mixture of the 15-mer and 6-mer libraries (4.5 x 10⁹ TTU each)- yield of tissue elution using Protocol N1 (Experiment E)**

| Round # | Input (TTU) | Output (TTU) | Yield (%) |
|---|---|---|---|
| R1E | 9x10⁹ | 3x10⁶ | 3x10⁻³ |
| R2E | 7x10¹⁰ | 2x10⁵ | 2x10⁻⁴ |
| R3E | 2x10¹⁰ | 1x10⁷ | 6x10⁻² |
| R4E | 1x10¹¹ | 4x10⁶ | 4x10⁻³ |
| R5E | 5x10¹¹ | 3x10⁸ | 6x10⁻² |

Ten clones were sequenced after R5E, i.e. the tissue eluted fraction from umbilical artery after round 5:
3/10 clones were identical to TUV-R4D-#3, which is also identical to YCA-4R4-#10 (Example 3).
1/10 clone was identical to TUV-R4B*-#3.
6/10 clones were unique clones.

**Table 9: Biopanning of umbilical artery with 15-mer library - yield of tissue elution using Protocol R2 (Experiment F).**

| Round # | Input (TTU) | Output (TTU) | Yield (%) |
|---|---|---|---|
| R1F | 5x10⁸ | 4x10⁶ | 7x10⁻¹ |
| R2F | 2x10¹⁰ | 6x10⁵ | 3x10⁻³ |
| R3F | 2x10¹⁰ | 2x10⁵ | 1x10⁻³ |
| R4F | 2x10¹⁰ | 1x10⁶ | 6x10⁻³ |
| R5F | 9x10⁹ | 4x10⁷ | 5x10⁻² |

Ten clones were sequenced after R5F, i.e. the tissue eluted fraction from umbilical artery after round 5:
8/10 clones were identical to clone TUV-R4B*-#1.
1/10 clone was identical to clone TUV-R4B*-#3 and 1/10 clone was a unique clone.

**Table 10: Biopanning of 15-mer library with umbilical artery with vein-excluded¹ phage library - yield of tissue elution using protocol N1 (Experiment H).**

| | Input (TTU) | Output (TTU) | Yield (%) |
|---|---|---|---|
| R1H | 1x10⁹ | 2x10⁵ | 2x10⁻² |
| R2H | 3x10⁹ | 8x10⁶ | 3x10⁻¹ |
| R3H | 6x10⁹ | 6x10⁷ | 9x10⁻¹ |
| R4H | - | 8x10⁶ | - |
| R5H | 8x10¹⁰ | 3x10⁷ | 3x10⁻² |

| | | | |
|---|---|---|---|
| ¹Phage library was first biopanned on the umbilical vein (8-10cm length), and only the unbound (vein-excluded) phage was then biopanned with umbilical artery (4-5 cm length). This sequence was repeated for all rounds. Tissue elution with starved bacteria was conducted according to protocol N1. | | | |

Seven clones were sequenced after R5H, i.e. the tissue eluted fraction from umbilical artery after round 5: 7/7 clones were identical to clone TUV-R4D-#3.

**Table 11: Binding of clone TUV-R4B*-#1 and clone TUV-R4B*-#3 (see section 3 below) to endothelial cell culture (Protocol EC-1); results of acid, tissue and urea elutions.**

| | Total colonies | clone | clone | clone | clone |
|---|---|---|---|---|---|
| | | TUV-R4B*- #1 (TTU) | TUV-R4B*- #1 (%) | TUV-R4B*- #3 (TTU) | TUV-R4B*-#3 (%) |
| input | 2x10⁹ | 3.4x10⁷ | 1.7 | 1.2x10⁷ | 0.6 |
| acid elution | 5x10⁵ | 7x10³ | 1.4 | 5x10³ | 1.0 |
| tissue elution | 3x10⁴ | 5.7x10² | 1.9 | N.D.¹ | N.D. |
| urea elution | 5x10⁴ | 5x10⁴ | 100 | N.D. | N.D. |

| | | | | | |
|---|---|---|---|---|---|
| ¹ N.D. = not detectable. | | | | | |

The results indicate that clone TUV-R4B*-#1 binds strongly to endothelial cells or to endothelial cell extra-cellular matrix, since 100% of the phages eluted with urea were identical to clone TUV-R4B*-#1.

### 3. Tissue distribution of TUV-R4B*-#1

In order to analyze tissue specificity of the selected phage displayed peptides, a phage mixture (4x10¹⁰) of a selected clone, either clone TUV-R4B*-#1 or clone TW-R4B*-#3 or both clones together, with the 15-mer library (in a ratio of 1:10- 1:100) was injected through the tail vein of a rat (in 0.5ml DMEM-1% BSA). After 4 minutes, the animal was sacrificed, the chest was opened, and extensive washing of the blood vessels was carried out by flushing isotonic salt solution through the left ventricle. After approximately 10 minutes, as the lungs became discolored, various organs were dissected, weighed, and homogenized in 1ml homogenization solution (DMEM-1%BSA-Pi) using an ultra thorax grinding device. Following centrifugation (10 minutes at top speed eppendorf centrifuge), the supernatant was discarded, and the pelleted tissue was washed three times with the same buffer. The washed tissue extract was resuspended in 0.6ml NZY and 0.4 ml starved bacteria (2x10¹⁰) and was incubated with gentle mixing for 10 minutes at 37°C. The tissue-bacterial suspension was diluted in 10ml NZY containing 0.2% tetracycline and after 45 minutes of vigorous mixing at 37°C, aliquots were plated on agar plates containing 40µg/ml tetracycline- 20µg/ml kanamycin and incubated at 37°c for 16 hours. After monitoring tet^{R} colonies on each plate, the colonies were transferred to a millipore sheet for colony hybridization as described in "Molecular Cloning: A Laboratory Manual", J, Sambrook, E.S. Fritsch, and T. Maniatis, Cold Spring Harbor Laboratory Press, 2nd edition, 1989.

Synthetic DNA probes of 24 and 23 oligonucleotides derived from the antisense sequence of clones TUV-R4B*-#1 and TUV-R4B*-#3 respectively, were used.

**Table 12: Tissue distribution of TUV-R4B*-#1 in a rat model (the ratio of clone TUV-R4B*-#1 to 15-mer library in the input phage was approximately 1:10).**

| | ¹Total No. of colonies | ²No. of TUV- R4B*-#1 | % of TUV- R4B*-#1 | % of 15-mer library |
|---|---|---|---|---|
| Input | 167 | 15 | 8.9 | 91.1 |
| Lung | 145 | 144 | 99.3 | 0.7 |
| Vena Cava³ | 70 | 47 | 67.1 | 32.9 |
| Aorta⁴ | 110 | 75 | 68.2 | 31.8 |
| Liver | 174 | 110 | 63.2 | 36.8 |
| Spleen | 108 | 58 | 53.7 | 46.3 |
| Brain | 204 | 130 | 63.7 | 36.3 |
| Kidney | 198 | 135 | 68.1 | 31.9 |

| | | | | |
|---|---|---|---|---|
| ¹ Total bacterial colonies on agar plates. ² Positive colonies after hybridization to the antisense radioactive probe of TUV-R4B*-#1. ³ Example of Vein. ⁴ Example of Artery. | | | | |

Tables 13A and 13B: Tissue distribution of clone TUV-R4B*-#1 and TUV-R4B*-#3 in rat model The ratio of TUV-R4B*-#1 and TUV-R4B*-#3 to the 15-mer library was approximately 1:1:98.

**Table 13A: Tissue distribution of TW-R4B*-#1**

| | ¹Total No. of colonies | ²No. of TUV- R4B*-#1 | % of TUV- R4B*-#1 | % of 15-mer library |
|---|---|---|---|---|
| Input | 800 | 16 | 1.1 | 98.9 |
| Lung | 9 | 8 | 88.8 | 11.2 |
| Vena Cava | 110 | 47 | 42.7 | 57.3 |
| Aorta | 249 | 106 | 42.5 | 57.5 |
| Liver | 230 | 152 | 66.0 | 33.0 |
| Spleen | 54 | 37 | 68.5 | 31.5 |
| Brain | 23 | 8 | 34.7 | 65.3 |
| Kidney | 22 | 12 | 54.5 | 45.5 |

| | | | | |
|---|---|---|---|---|
| ¹ Total bacterial colonies on agar plates. ² Positive colonies obtained by hybridization to the antisense radioactive probe of TW-R4B*-#1. | | | | |

The results of tables 12 and 13A demonstrate that clone TUV-R4B*-#1 is enriched in the rat lung, i.e. has highest specificity to the lung. Therefore, this peptide can be used as a specific marker for *inter alia* lung tissue, for drug delivery to lung tissue and to image lung tissue.

**Table 13B: tissue distribution of clone TUV-R4B*-#3**

| | ¹Total No. of colonies | ²No. of clone TUV- R4B*-#3 | % of TUV- R4B*-#3 | % of 15-mer library |
|---|---|---|---|---|
| Input | 484 | 4 | 0.82 | 99.1 |
| Lung | 60 | 10 | 16.6 | 83.4 |
| Vena Cava | 53 | 31 | 5.8 | 94.2 |
| Aorta | 416 | 41 | 9.8 | 90.2 |
| Liver | 204 | 59 | 28.9 | 71.1 |
| Spleen | 38 | 7 | 18.4 | 81.6 |
| Brain | 29 | 5 | 17.2 | 82.8 |
| Kidney | 37 | 8 | 21.6 | 78.4 |

| | | | | |
|---|---|---|---|---|
| ¹ Total bacterial colonies on agar plates. ² Positive colonies by hybridization to the antisense radioactive probe of TUV-R4B*-#3. | | | | |

### EXAMPLE 2: Isolation and Selection of Peptide Epitopes which Specifically Bind to Undetermined Targets in Safenal Vein, Umbilical Artery and Radial Artery.

As described in Example 1, a 6-mer phage display peptide library was kindly provided by G. Smith (Virology 167: 156-165, 1988). The library was amplified to form a phage library working stock.

The following protocol was used for the identification of peptides that specifically bind to
(1) safenal vein (JSV1);
(2) umbilical artery (JUA1); and
(3) radial artery (JRA1).

The 6-mer bacteriophage library displayed on the pill coat protein was used, and the selection procedure was carried out by perfusion for 4 or 5 rounds of biopanning essentially as described for Protocol R1 and R2 in Example 1.

Before panning with the phage library, blood vessels were prewashed by perfusing cold (4°C) DMEM-5% human serum/5U/ml heparin solution.

Following *ex-vivo* perfusion of the phage library (in the first round approximately 2x10¹² phages were used), non-specifically bound phages were extensively washed with DMEM 5% human serum containing a mixture of protease inhibitors (Pi). Tightly bound phages were eluted at room temperature sequentially with 2ml trypsin 0.25%/EDTA 0.05% solution and then with 5ml acid (0.2M glycine-HCl pH-2.2) solution, containing 0.5% Tween (Protocol R1). The recovered phages from both washes were combined, neutralized, titered and amplified. Although the above treatment was sufficient to recover most of the bound phage, some phages remained associated with tissue. This tissue associated fraction was also recovered by displacement with prestarved *E. coli* cells perfused into the acid washed blood vessels at 37°C for 1 hour (Protocol R2). The tissue fraction was titrated and amplified as a separate stock for each blood vessel [tissue wash safenal vein (JTSV1), tissue wash umbilical artery (JTUA1) and tissue wash radial artery (JTRA1), respectively] .

In the subsequent rounds of phage amplification, the experiments were carried out essentially as described above, except that approximately 2x10¹⁰ phages were used for each cycle of biopanning, while maintaining tissue and fraction specificity (for example, when an acid-eluted fraction of the phage was initially recovered (R1), then only the acid fraction was amplified in all subsequent rounds).

After four to five rounds of biopanning there was an enrichment in the recovered phages by at least 3 orders of magnitude compared to the yield obtained after the first round of panning.

To examine the effect of the above treatments (including the wash with trypsin/acid/tween as described in protocol R1 in Example 1) on the endothelial cell layer which line the inner side of the vessel wall, histological sections were prepared. No major changes were observed in the endothelial cells surrounding the lumen of the perfused blood vessels compared to non-treated vein or artery.

After the selection and enrichment procedure, single stranded DNA was prepared for sequencing from individual colonies of phage-infected bacteria. After five rounds of amplification of the safenal vein acid elution phage fraction, twenty-two clones were sequenced. Apart from one peptide sequence designated JSV1-#13, which appeared twice (9%), none of the phage peptide sequences were identical.
**Amino acid sequence displayed by clone JSV1-#13**

Phe Leu Pro Asn Gly Phe

1 2 3 4 5 6

In contrast, after four rounds of amplification, five of seven (71%) safenal tissue associated phages were identical. The peptide encoded by these phages was designated JTSV1-#7. The other two clones designated JTSV1-#26 were also identical.
**Amino acid sequence displayed by clone JTSV1-#7** 
Thr His Asp Thr His Leu
1 2 3 4 5 6
**Amino acid sequence displayed by clone JTSV1-#26**

Thr His Glu Thr Gln Arg
1 2 3 4 5 6

All six clones isolated after umbilical artery selection from the acid elution fraction were different. One of these peptides (16%), was designated JUA1-#1. This amino acid sequence was also isolated once (1/7, 14%) from the tissue associated umbilical artery (JTUA1) phage stock and once (1/22, 4.5%) from the tissue associated safenal vein (JTSV1) phage stock.
**Amino acid sequence displayed by clone JUA1-#1**

Asp Ile Ala Lys Arg Tyr
1               5

Interestingly, after four rounds of biopanning, the amino acid sequence of JTSV1-#7, was also recovered from JTUA1 (tissue associated umbilical artery) amplified phage stock with an identical frequency (5/7, 71%) as from JTSV1 (tissue associated fraction). Moreover, seven of seven clones (100%) of JTRA1 enriched fraction were identical to JTSV1-#7. The other two clones in the JTUA1 tissue associated fraction were different. This result suggests that clone JTSV1-#7 binds specifically to a general target in the blood vessel, regardless of its source.

In an attempt to eliminate the binding of JTSV1-#7 to artery, the 6-mer library (5x10¹⁰TTU) was first passed through the umbilical vein and the unbound phages were passed directly through the umbilical artery. The protocol for the biopanning, washing and elution was basically as described for the tissue associated fraction in the first set of experiments (for example JTUA1)

Following four rounds of biopanning, the tissue associated amplified phage stock was designated JTUVA2. Single stranded DNA was prepared from fourteen random clones. Thirteen of fourteen (13/14, 93%) clones isolated from the JTUVA2 phage stock were identical to clone JTSV1-#7.

Organ and cell binding specificities of clones JTSV1-#7, JSV1-#13 and JTSV1-#26 were each evaluated by biopanning separately in a mixture with the 6-mer initial stock library. The phage binding specificity to several organs removed from mice injected into the tail vein with phage mixture is shown in Table 1. Binding to human blood vessels perfused *ex vivo* is shown in Table 2.

The specific phage binding frequency was detected by hybridization with a radioactive antisense oligonucleotide complementary to the sequence encoded by the corresponding phage.

**TABLE 1**

| Tissue distribution of clones JTSV1-#7, JTSV1-#26 and JSV1-#13 in mouse model. The input was 0.1% of each clone within 10¹⁰TTU 6-mer library). | | | |
|---|---|---|---|
| | **JTSV1-#7** | **JSV1-#13** | **JTSV1-#26** |
| Heart | Binds at a high frequency to all organs | 3% | 24% |
| Spleen | | 1% | 2% |
| Brain | | 3% | 0% |
| Lung | | 5% | 0% |
| Kidney | | 12% | 0% |
| Liver | | 1% | 0% |

**TABLE 2**

| Binding specificity of clones (5% each within 10¹⁰ 6-mer library) to umbilical cord (artery or vein) | | | | | |
|---|---|---|---|---|---|
| **Input clone (5%)** | | **Umbilical Artery** | **Umbilical Vein** | **Mammary Artery** | **Safenal Vein** |
| JTSV1-#7 | | >80% | >80% - | - | - |
| JSVI-#13 | | 13% | 25% | - | - |
| JTSVI-#26 | | 20% | 80% | - | - |
| (Mixture of | - Hybridization with JSV1-#13 | 0.1% | 28% | 10-20% | 0.1% |
| JSV1-#13 and JTSV1-#26 | - Hybridization with JTSV 1-#26 | 0.2% | 50% | 0.1% | 20% |

The specific hybridization assays show that clone JTSV1-#26 is vein specific, whereas clones JTSV1-#7 and JSV1-#13 bind vein and artery at about the same affinity (see Table 2).

### EXAMPLE 3: Isolation of peptides which specifically bind to undetermined targets in rat coronary artery

### Peptide display phage library source

As described in Example 1, a 15-mer phage display peptide library was kindly provided by G. Smith (Virology 167: 156-165, 1988). The library was amplified to form a phage library working stock.

### Preparation of perfused rat coronary arteries

Isolated rat hearts were subjected to a Langendorff-type perfusion system, as described by Neely & Rovetto (1975), Methods in Enzymology 39: 43-60), using a modified Krebs-Henzeleit bicarbonate buffer, pH 7.4, equilibrated with O₂:CO₂(95:5) at 37°C as the basic perfusion medium. The system consisted of a 90 cm glass jacketed condenser, in which a constant 90 cm head of the medium was kept (by use of an overflow system). The temperature was kept constant by a flow of thermostated water through the jacket, and equilibration with the gas mixture was assured by slow bubbling of the gas at the bottom of the inner section of the condenser, facilitated through a narrow polyethylene tube inserted from the top. A three-way stopcock was connected between the outlet of the condenser and the heart cannula (made of a 18-gage needle), and an infusion pump was connected to the side arm of the stopcock using a narrow polyethylene tubing (thus keeping a minimal dead volume).

After connection of the organ to the cannula, the buffer was allowed to flow freely into the heart, thus facilitating regular beating. The flow rate (7-12ml/min) was then measured, followed by an initiation of a sidearm flow of 5% BSA in the same buffer at a 1/9th rate, thus resulting in a final concentration of BSA of 0.5% in the heart perfusate. The hearts were perfused at these conditions for 10 min. The three-way valve was then adjusted to flow through the sidearm only, and a suspension of the phages (10¹⁰-10¹² phages) in 20 ml of 0.5% BSA in perfusion buffer was introduced into the heart at a flow rate of 4ml/min. The free flow of perfusion buffer + BSA 0.5% was resumed for an additional 10 minutes. At the end of the wash period, the valve was again adjusted to a sidearm flow only, and 0.1 ml of a solution of Trypan Blue (% in) was injected into the heart vasculature, in order to clearly visualize the coronary arteries. The heart was then removed, and a piece of tissue, consisting mainly of the coronary artery, was excised and taken for homogenization.

### Bacterial strain

The bacterial strain used was as described above in Example 1.

### Biopanning protocols

Several biopanning protocols were developed:
A. Protocol YMCA-1
B. Protocol YMCA-2
C. Protocol YMCA-4
D. Protocol YMCA-5

### Protocol YMCA-1

1. Prewash: the heart vasculature was washed in Krebs-Henzeleit bicarbonate buffer (perfusion buffer), 0.5%BSA through the aorta for 10 minutes, at a flow rate of 8-12 ml/min.
2. Ex vivo selection was carried out at 10¹⁰-2x10¹¹ TTU in 20 ml perfusion buffer, 0.5% BSA-Pi at a flow rate of 4 ml/min for 5 min at 37°C.
3. Wash was carried out in perfusion buffer, 0.5%BSA for 10 min at 8-12 ml/min.
4. Elution: The coronary artery was excised and gently grounded in 0.3 ml DMEM, 0.5%BSA-Pi. The tissue was washed with 0.3 ml DMEM, 0.5%BSA-Pi. Tissue-associated phages were recovered at room temperature during 1 hour, by displacement with 2 x 10⁹ starved *E.coli* cells in 0.3 ml.

**TABLE 1: Biopanning of 15-mer library in rat coronary artery (protocol YMCA-1)**

| BIOPANNING ROUND | INPUT | OUTPUT | TISSUE-ASSOCIATED PHAGE (%) |
|---|---|---|---|
| YMCA-1, R-1 | 10¹⁰ | 6x10⁴ | 6x10⁻⁴ |
| YMCA-1, R-2 | 2x10¹¹ | 8x10⁴ | 4x10⁻⁵ |
| YMCA-1, R-3 | 2x10¹⁰ | 1.2x10⁵ | 6x10⁻⁴ |
| YMCA-1, R-4 | 10¹¹ | 9.6x10⁵ | 10⁻³ |
| YMCA-1, R-5 | 2x10¹¹ | 3.6x10⁷ | 1.2x10⁻² |

The results of this experiment demonstrated a 100 fold increase of tissue-associated phage between the first and fifth round (R) of biopanning.

The DNA sequence of seven independent tissue-associated phages was determined after round 5. Three of these clones were designated YCA-1R5-#11, YCA-1R5-#2 and YCA-1R5-#13.
**Amino acid sequence displayed by clone YCA-1R5-#11**

Arg Asn Val Pro Pro Ile Phe Asn Asp Val Tyr Trp Ile Ala Phe
1               5                   10                  15

Clone YCA-1R5-#11 is identical to clone TUV-R5D-#2 selected independently from umbilical vein in Example 1.
**Amino acid sequence displayed by clone YCA-1R5-#2** 
Asp Val Ser Pro Val Trp Ala Ala Phe Ala Ser Gly Ala Ser Phe
1 2 3 4 5 6 7 8 9 10 11 12 13 14 15
**Amino acid sequence displayed by clone YCA-1R5-#13**

Ser Leu Trp Gly Ala Ser Ser Cys Gly Val Ala Phe Phe Glu Ser

1 2 3 4 5 6 7 8 9 10 11 12 13 14 15

### Protocol YMCA-2

1. Prewash was carried out as in protocol YMCA-1.
2. Selection was carried out as in protocol YMCA-1
3. Wash was carried out as in protocol YMCA-1
4. Elution: the coronary artery was excised and gently grounded by homogenizer (KIKA-WERK) (1000 rpm) in 0.3 ml DMEM, 0.5%BSA-Pi. The tissue was washed in 0.3ml 0.13M glycine-HCl, pH2.2 for 5 minutes, neutralized in 21µl Trisma base, washed in 0.3ml DMEM, 0.5%BSA-Pi and tissue-associated phages were recovered at room temperature for 1 hour by displacement with 2 x 10⁹ starved *E.coli* cells in 0.3 ml.

**TABLE 2: Biopanning of 15-mer library with rat coronary artery (protocol YMCA-2)**

| BIOPANNING ROUND | INPUT | OUTPUT | TISSUE-ASSOCIATED PHAGE (%) |
|---|---|---|---|
| YMCA-2, R-1 | 10¹⁰ | 4.6x10⁴ | 4.6x10⁻⁴ |
| YMCA-2, R-2 | 5x10¹¹ | 2.5x10⁵ | 5x10⁻⁵ |
| YMCA-2, R-3 | 10¹⁰ | 1.6x10⁴ | 1.6x10⁻⁴ |
| YMCA-2, R-4 | 10¹¹ | 10⁵ | 1x10⁻⁴ |
| YMCA-2, R-5 | 10¹¹ | 10⁵ | 1x10⁻⁴ |

7/7 clones from round 5 were identical to clone YCA-4R4-#10 (see YMCA-4 below).

### Protocol YMCA-4

1. Prewash was carried out as in protocol YMCA-1.
2. Selection was carried out as in protocol YMCA-1
3. Wash was carried out as in protocol YMCA-1
4. Elution: the coronary artery was excised, washed in 0.3ml 0.13M glycine-HCl pH2.2 for 5 minutes and neutralized with 21*µ*l Trisma base. The tissue was then ground vigorously in 0.3 ml DMEM, 0.5%BSA-Pi and tissue-associated phages were recovered at room temperature for 1 hour by displacement with 2 × 10⁹ prestarved *E.coli* cells in 0.3 ml.

**TABLE 3: Biopanning of 15-mer library with rat coronary artery (protocol YMCA-4)**

| BIOPANNING ROUND | INPUT | OUTPUT | TISSUE-ASSOCIATED PHAGE (%) |
|---|---|---|---|
| YMCA-4, R-1 | 10¹⁰ | 2.2x10⁵ | 2.2x10⁻³ |
| YMCA-4, R-1 | 10¹⁰ | 8x10⁵ | 8x10⁻³ |
| YMCA-4, R-2* | 3×10¹⁰ | 1x10⁶ | 3x10⁻³ |
| YCA-4, R-3 | 10¹¹ | 2.4x10⁶ | 2.4x10⁻³ |
| YMCA-4, R-4 | 10¹¹ | 1.4x10⁷ | 1.4x10⁻² |
| YMCA-4, R-5 | 2×10¹¹ | 3.8x10⁷ | 1.9x10⁻² |

| | | | |
|---|---|---|---|
| * The input phage of R-2 was a mixture of the output of both R-1's. | | | |

The results of this experiment demonstrated a 100 fold increase between the first and the fourth round of panning. There was no significant increase between the fourth and fifth round of panning.

Ten clones were sequenced after the fourth round:
(i) 4/10 (40%) clones were identical (clones 6,7,9,10) to clone YCA-4R4-#10 (see YMCA-2).
   Amino acid sequence displayed by clone YCA-4R4-#10:

Gly Cys Gly Gly Phe His Pro Gly Ser Asn Cys Leu Val Val Ser
1 2 3 4 5 6 7 8 9 10 11 12 13 14 15

   Because of the cysteines in positions 2 and 11, this clone may be a cyclic peptide. This sequence is positively charged (+1).
   In addition, a similar sequence to Gly Cys Gly Gly (underlined) was found in clone YCA-4R4-#3 (see below) which comprises the sequence Gly Leu Gly Gly. Therefore, Gly X Gly Gly may be a consensus binding sequence, wherein X is Cys or Leu or any other amino acid.
   Moreover, the sequence Asn Cys Leu (underlined) also appeared in clone YCA-4R4-#5 (see below) and thus, this sequence may be a consensus binding sequence.
   Furthermore, clone YCA-4R4-#10 is identical to clone TUV-R4D-#3 selected independently in veins of human umbilical cord, as disclosed in Example 1.
(ii) 2/10 (20%) clones were identical to YCA-1R5-#2.
(iii) 4/10 (40%) clones were unique. These clones were designated YCA-4R4-#5, YCA-4R4-#4, YCA-4R4-#3 and YCA-4R4-#8.
   **Amino acid sequence displayed by clone YCA-4R4-#5**

Ser Asp Cys Leu His Ser Val Arg Gly Phe Asn Cys Leu Lys Arg
1 2 3 4 5 6 7 8 9 10 11 12 13 14 15

   Clone YCA-4R4-#5, although a unique clone, is interesting because it has the motif Asn Cys Leu which was also found in clone YCA-4R4-#10. Furthermore, clone YCA-4R4-#5 has cysteine residues in positions 3 and 12 and thus the displayed peptide may be cyclic. Clone YCA-4R4-#5 has a positive charge of +3.
   **Amino acid sequence displayed by clone YCA-4R4-#4** 
Ser Leu Cys Phe Tyr Leu Phe Val Met Ser Ala Pro Asp Ala Pro
1 2 3 4 5 6 7 8 9 10 11 12 13 14 15
   **Amino acid sequence displayed by clone YCA-4R4-#3**

Gly Leu Gly Gly Leu Ser Phe Gly His Ser Asp Asn Pro Pro Ser
1 2 3 4 5 6 7 8 9 10 11 12 13 14 15

   The sequence Gly Leu Gly Gly (underlined) is of interest because a similar sequence appeared in clone YCA-4R4-#10 as discussed above.
   **Amino acid sequence displayed by clone YCA-4R4-#8**

Gly Pro Gly Trp Val Gly Trp Phe Val Ser Leu Phe Tyr Ala Ser
1 2 3 4 5 6 7 8 9 10 11 12 13 14 15

   In addition, seven clones were sequenced after the fifth round. One of these clones was designated YCA-4R5-#12.
   **Amino acid sequence displayed by clone YCA-4R5-#12:**

Trp Ser Leu Gly Ser Ser Trp Val Tyr Lys Phe Phe Tyr Ser Ser
1 2 3 4 5 6 7 8 9 10 11 12 13 14 15

### Protocol YMCA-5 - In vivo selection

To select peptides *in vivo* that specifically bind to the coronary artery, 2×10¹¹ TTU of amplified phage eluate from YMCA-4, R-4 diluted in 0.5ml DMEM from YMCA-4, R-4 was injected intravenously to live rats (into the tail vein).
After 5 minutes animals were anesthetized and subsequently sacrificed. The heart was removed and washed with perfusion buffer 0.5% BSA for 10 minutes at 8-12 ml/min. The coronary artery was isolated, excised and the bound phages were eluted as described above for protocol YMCA-4.

Phages were recovered from the coronary artery, titered, amplified and sequenced.

**TABLE 4: In vivo biopanning on rat coronary artery (protocol YMCA-5)**

| BIOPANNING ROUND | INPUT | OUTPUT | TISSUE-ASSOCIATED PHAGE (%) |
|---|---|---|---|
| YMCA-5, R-1 | 2x10¹¹ | 5x10⁵ | 2.4x10⁻⁴ |

DNA of seventeen clones were sequenced, three of which were identical to YCA-1R5-#11.

This experiment was performed together with YMCA-4, R-5 as a control. The input in both experiments was amplified eluate YMCA-4, R-4, 2x10¹¹ TTU.

Tables 3 and 4 demonstrate that the output of clone YMCA-5, R-1 was 100 fold less (5x10⁵) then the output of clone YMCA-4, R-5 (3.8x10⁷) .

### EXAMPLE 4: Preparation of a peptide

A peptide of the subject invention, corresponding to the sequence of a peptide displayed by a phage virion, is prepared by the following methods:

### I. Chemical (organic) synthesis

Chemical synthesis of peptides is carried out by methods well-known in the art, e.g. solid phase synthesis of Merrifield, J. Amer. Chem Soc. 85: 2149-2154 (1963); Science 150: 178-185 (1965); Ibid 232, 341-347 (1986). The solid phase synthesis provides a growing peptide chain anchored by its carboxyl terminus to a solid support, e.g. a resin such as chloromethylated polystyrene resin or p-methylbenzhydrylamin resin when synthesizing a peptide amide derivative. The use of various N-protecting groups, e.g. the carbobenzyloxy group, the t-butyloxycarbonyl group (BOC) or the N-(9-fluorenyl-methylcarbonyl) group (Fmoc), various coupling reagents, e.g. dicyclohexylcarbodiimide (DCC) or carbonyldiimidazole, various cleavage reagents, e.g. trifluoracetic acid (TFA) in methylene chloride (CH₂Cl₂) and other such reagents of classical solution phase peptide synthesis are also used in conventional solid phase synthesis of peptides.

Various commercial companies prepare peptides custom-made, i.e. specifically as ordered by customers. Examples of such companies are Bio-Synthesis, Research Genetics, Inc., and AnaSpec (Science 275: 270 (1997)).

The chemically synthesized peptide is linked to a pharmaceutical agent (drug) *inter alia* by a covalent or by a non-covalent bond forming a drug-peptide conjugate. The peptide may also be incorporated into a liposome. The covalent bond may be *inter alia* a peptide, an amide, an ester, a disulfide or an anhydride covalent bond. The non covalent bond is *inter alia* an ionic bond or a hydrophobic complex. The pharmaceutical agent may be *inter alia* a radio isotope label.

The choice of the peptide linkage is determined based on the functional groups of the individual drug.

The peptide may also be cyclic (see e.g. Example 3). Cyclic peptides having a disulfide bond may be prepared as described in U.S. Patent No. 4,903,773 (Partoliano and Ladner) .

### II. Recombinant technology

Recombinant production of peptides is carried out by methods known in the art. The DNA encoding the peptide is prepared by synthetic oligonucleotides based on the amino acid sequence of the peptide and their known nucleotide codons (see e.g. U.S. Patent No. 5,221,619). The peptide is then produced by expression of the nucleotide sequence encoding the polypeptide.

The recombinantly produced peptide is linked to a pharmaceutical agent. When the pharmaceutical agent is a recombinant polypeptide, a hybrid fusion polypeptide is constructed comprising the peptide linked by a peptide bond to the drug as follows: a DNA molecule is prepared comprising DNA encoding the drug and DNA encoding the peptide. The DNA encoding the peptide is prepared by synthetic oligonucleotides based on the amino acid sequence of the peptide and their known nucleotide codons. The synthetic oligonucleotide encoding the peptide is ligated either to the 5' end of the DNA strand encoding the drug or to the 3' end. When ligated to the 5' end of the DNA encoding the drug, an ATG nucleotide sequence is added to the synthetic oligonucleotide encoding the peptide. When ligated at the 3' end of the DNA encoding the drug, a DNA termination codon (TAA or TGA) is added at the 3' end of the synthetic oligonucleotide encoding the peptide and the termination codon at the 3' end of the DNA encoding the drug is removed using conventional recombinant DNA technology methods by use of Mung bean nuclease or cleavage with an appropriate restriction enzyme.

The hybrid polypeptide encoded by the above described recombinant DNA molecule is expressed and produced by recombinant technology by methods known in the art, e.g. in bacteria, yeast, insect, plant or mammalian cells in culture or in a genetically engineered transgenic animal or plant.

### III. Computer-aided design technology

In recent years, intensive efforts have been made to develop peptidomimetics or peptide analogs that display more favorable pharmacological properties than their prototype native peptides. The native peptide itself, the pharmacological properties of which have been optimized, generally serves as a lead for the development of these peptidomimetics. A lead structure for development of a peptidomimetic can be optimized, for example, by molecular modeling programs.

Computer-aided design technology known in the art is used in order to produce a mimotope, i.e. a peptide mimetic of a peptide of the subject invention, the chemical structure of which is different from the peptide, but the biological activity of which remains similar to that of the peptide. U.S. Patent Nos. 4,879,313, 4,992,463 and 5,0191,396 describe examples of such peptide mimetic compounds.

To the peptide of the subject invention, produced by any one of the above three methods, amino acid residues may be added, deleted or substituted using established well known procedures, thereby producing extended peptides.

Furthermore, the DNA encoding the peptide of the subject invention may be mutated by methods known to those skilled in the art, thereby produced mutated peptides.

As mentioned above, the peptide of the subject invention, produced by any one of the above three methods, may be administered to a patient, alone, radiolabeled, or linked to a pharmaceutical agent (drug).

The following pharmaceutical agents are examples of drugs which may be linked to a peptide of the subject invention corresponding to a peptide displayed by a phage virion to form drug-peptide conjugates. Other pharmaceutical agents not mentioned below may also be used.

The peptide of the subject invention may be linked to a toxin, an anti-cancer drug or an anti-angiogenic compound in order to target and destroy tumor tissue. This specific use of such drug-peptide conjugates represents an alternative approach to current attempts to apply immunotoxins against cancer. Solid tumor growth *in vivo* is associated with recruitment of new blood vessels. Targeting the tumor-vasculature is an attractive possibility for anti-cancer therapy for the following reasons: First, most anti-cancer drugs are given by the systemic intravenous route and drug concentration at the tumor tissue is the crucial factor for effective therapy. Secondly, one limitation of current anti-cancer therapy is toxicity. Targeting the drug by cross-linking with a tissue specific peptide circumvents the toxicity problem. Thirdly, targeted anti-vascular or anti-angiogenesis therapy obliterates the capacity of the tumor to continue to grow and metastize as blood supply of fresh nutrient is blocked.

Examples of anti-cancer drugs that can be linked to the peptide of the subject invention are adriamycin, cis-Platinum, taxol, bleomycin and so forth.

Examples of anti-angiogenic compounds that can be linked to peptide of the subject invention are cortisone, heparin and so forth.

Examples of toxins that can be linked to peptide of the subject invention are *Pseudomonas* exotoxin A, ricin and so forth.

Several major drugs applied in heart conditions are given by the systemic intravenous route while their target, the coronary arteries, constitute only a small portion of the vasculature. Therefore, targeting to the coronaries or even to arteries in general concentrates the drug to the diseased vessels and reduces undesired side effects.

Examples of cardiovascular drugs that can be linked to the peptide of the subject invention are thrombolytic enzymes such as tissue plasminogen activator (tPA), streptokinase (SK), and anti-thrombotic agents such as heparin, ticlopidine or antiplatelet monoclonal antibodies.

The peptide of the subject invention can be further linked to recombinant proteins such as CuZnSOD, MnSOD, Factor Xa Inhibitors, erythropoietin, von Willebrand Factor or fragments thereof, ecto-enzymes such as Apyrase and so forth. Such recombinant proteins are described *inter alia* in U.S. Patent Nos. 5,126,252 and 5,360,729 (CuZnSOD), U.S. Patent Nos. 5,270,195 and 5,246,847 (MnSOD), WO 91/01416 (von Willebrand factor fragment), WO 94/03871 (Factor Xa Inhibitor), and U.S. Patent No. 4,703,008 (erythropoietin).

The most common brain disorders include tumors, stroke, head trauma, epilepsy, infectious agents, Parkinson's disease and Alzheimer's disease. Several drugs have been approved for these indications. However, there is a need to increase the level of drug reaching the brain vasculature and developing a delivery system which transports therapeutics across the blood-brain barrier (BBB).

Examples of "CNS drugs" that may be linked to the peptide of the subject invention are L-Dopa, Cortisone, tPA or phenobarbital.

Both the liver and the kidney are suitable for targeted drug delivery via the vascular system since they are well perfused tissues, involved in the metabolism of many endogenous and exogenous compounds. Targeting of liver and kidney is important particularly for cancer therapy and hepatitis B and C virus infection of the liver.

Examples of drugs for use in the treatment of liver and kidney disease that can be linked to the peptide of the subject invention are anti-viral drugs such as Interferon, Iododeoxyuridine, and Adenine arabinoside. Adriamycin can be used for the treatment of renal cancer and Glucocerebrosidase 6-thioguanine for the treatment of Gaucher's disease.

Upon injection, a recombinant protein is targeted to tissue specific endothelial cells and is converted from a soluble protein to a cell surface bound protein (ectoenzyme). In this manner, arteriosclerosis and thrombogenicity of the vessel wall in certain clinical indications is reduced. Conversion of these soluble proteins into ectoenzymes also reduces blood clearance time and hence a lower amount of recombinant protein is injected to achieve efficacy.

### EXAMPLE 5: Selection of Tissue-Specific Epitopes which Preferentially Bind to a Damaged Artery

### Biopanning of a damaged human artery with a phage peptide library.

Local damage is made in an artery by using the current methodology of angiography, PTCA (percutaneous transluminal coronary angiography) ballooning. Upon insertion of the inflated balloon, denudation of blood vessel endothelial cells occurs and the subendothelium is exposed. The exposed subendothelium is thrombogenic and causes post PTCA reocclusion and restenosis.

Displayed peptides having specific amino acid sequences are obtained by biopanning the damaged artery with a phage display peptide library. Phages that specifically bind the exposed subendothelium (but not the normal ("healthy") blood vessel endothelial cells) are isolated and the DNA sequence is determined in order to obtain the corresponding amino acid sequence of the displayed peptide.

The peptide corresponding to the displayed peptide sequence (or a peptide mimetic thereof) is linked to an anticoagulant and/or anti-antithrombotic agent and/or anti-arteriosclerotic agent as described in Example 4. The peptide or peptide-drug conjugate is used to target exposed subendothelium and prevent platelet aggregation, thrombus formation, restenosis and arteriosclerosis.

### EXAMPLE 6: Isolation of tumor specific endothelium epitopes by ex vivo perfusion of a phage display library through a solid tumor vascular system derived from human colon lung, kidney, liver and ovary carcinoma tissues

Human colon, lung, kidney, liver and ovary carcinoma tissues obtained after surgery are each separately perfused *ex vivo* via their vascular system, and biopanned with a phage peptide library according to the protocols described in Examples 1, 2 and 3.

Phage clones displaying unique peptide sequences are isolated. These clones bind specifically to an above referenced diseased organ, but not to the healthy corresponding organ.

The amino acid sequences of these unique displayed peptides are determined by sequencing the corresponding DNA of the phage. The corresponding peptides are synthesized as described in Example 4. These peptides (or peptide mimetics thereof) are used, alone or linked to anti-cancer agent, as anti-metastatic and anti-cancer agents.

### EXAMPLE 7: Biopanning of T lymphocytes from Chronic Lymphocytic Leukemia patients (T-CLL)

Lymphocytes derived from patients suffering from leukemia or lymphoma differ from normal, healthy derived lymphocytes as they express different or modified epitopes on their surface.

### Antibody library source

A human antibody synthetic library (Nissim-N1 library, EMBO J., 13:692, 1994) was provided by Dr. Ahuva Nissim. The diverse repertoire of rearranged V_{H} genes was built in-vitro by PCR from a bank of human V_{H} gene segments derived from 49 individual germlines and random nucleotide sequence encoding CDR3 lengths of 4-12 residues. The amplified rearranged V_{H} fragments were cloned with a single unmutated V3 segment derived from the germline IGLV3S1 (isolated from an antibody binding to BSA), as a single chain Fᵥ fragment for phage display. This procedure created a single pot phagemid library of more than 10⁸ different clones and is displayed on the N-terminus of the pIII of the M13 bacteriophage.

### Panning strategy and results

Lymphocytes isolated from the peripheral blood of T-CLL patient were biopanned with the single pot phagemid antibody library as follows: For the first round of selection, approximately 10⁷ T-CLL cells were washed, blocked with PBS containing 4% skim milk and M13 and panned with 10¹² phagemids-from the N1 library, in the presence of wild-type M13 (10¹³). After incubation at 4°C for 1hr with slow agitation, cells were washed four times with PBS/milk, and the bound phagemids were eluted from the cells at room temperature for 5 min with 0.1M glycine pH-2.2. After neutralization, cells were spun, discarded, and the supernatant containing the eluted phagemids was collected and was designated E1 stock.

The subsequent rounds were performed with 2×10⁶ T-CLL cells.
a) The phagemids in E1 output were amplified by infecting E. coli bacteria (TG-1 strain) and plating them on ampicillin agar plates. Phagemids were rescued by superinfecting exponentially growing amp^{r} bacteria with helper phage (VSC-M13, Stratagene). This amplified stock-E1 was used for three additional rounds of panning, except that no amplification step was involved after each round of panning. After the last round of panning, the output phagemid was amplified and designated E4/1. Phagemids binding at high rate to T-CLL cells were characterized by DNA sequencing.
   Twenty phagemid clones picked randomly from the E4/1 output were grown individually and a phagemid stock was prepared. These phagemid clones were mixed at a 1:1 molar ratio and used in three sequential rounds of panning on T-CLL cells and on normal blood lymphocytes (NBL).
   All the phagemid clones tested which are derived from the second and from the third output were identical and code for a 10-mer CDR3 insert, whereas all 14 clones derived from second panning of the NBL were different from each other, and also not similar to that obtained in the T-CLL outputs. The repeating clone obtained in this procedure was designated JTCLL-IC#2. DNA sequencing of this clone was performed.
   **Amino acid sequence displayed in the CDR3 of clone JTCLL-IC#2:** 
Arg Ser Lys Tyr Arg Pro Asn Met Thr Asn
1 2 3 4 5 6 7 8 9 10
b) The amplified E1 phagemid stock was used for two sequential rounds of panning as above, except that after washing, the bound phagemids were eluted for 15 min at room temperature with either PBS containing 10mM EDTA (E1ED2 stock) or with PBS containing 10mM ATP (E1AT2 stock).
   DNA of phagemid clones derived from the ATP elution-E1AT2 stock (15 clones) and from the EDTA elution-E1ED2 stock (22 clones) were sequenced. Clones JTCLL-EDAT#10 and JTCLL-EDAT#22 appeared in both stocks E1AT2 and E1ED2 in 4/ 37 and 5/37 of the tested clones, respectively. The CDR3 encoded by these repeating clones is as follows:
   **Amino acid sequence displayed in the CDR3 of clone JTCLL-EDAT#10:** 
Leu Asn Pro Lys Val Lys His Met
1 2 3 4 5 6 7 8
   **Amino acid sequence displayed in the CDR3 of clone JTCLL-EDAT#22:** 
Leu Arg Gly Gly Asn Ala Met
1   2   3   4   5   6   7
c) Prior to amplification, the E1 output was used for two additional rounds of panning. The output phagemids were then amplified as above to give E3 stock. This E3 amplified stock (derived from only 300 different colonies) was used for three additional sequential rounds of panning and then amplified (E3CIII stock). In parallel, the last procedure was carried out also with NBL. After infection, the output from this procedure was designated **E3NIII**.
   Clones derived from E3CIII and E3NIII outputs were sequenced: (i) all 23 clones tested from the E3NIII output were identical and designated JTCLL-T#29 (ii) in the E3CIII output 9/21 were identical to JTCLL-T#29 and 5/21 clones were designated JTCLL-E3C#2.
   **Amino acid sequence displayed in the CDR3 of clone JTCLL-T#29:** 
Val Ser Asp Arg Arg Gln Asn Val
1 2 3 4 5 6 7 8
   **Amino acid sequence displayed in the CDR3 of clone JTCLL-E3C#2:**

Ser Lys Ser Pro
1 2 3 4

All peptides corresponding to the displayed peptides are synthesized as described in Example 4. These peptides (or peptide mimetics thereof), alone or linked to anti-cancer agent or to a radioisotope, are used as anti-cancer agents or for tumor diagnostics.

### EXAMPLE 8: Biopanning of human lymphocytes for predetermined targets (CD44 variants) with a phage peptide library

### Background

Numerous adhesion molecules are expressed by tumor cells and a variety of adhesive interactions are mediated by them. These adhesion molecules act as both positive and negative modulators of invasive and metastatic processes. One of these adhesion proteins is CD44, a transmembrane glycoprotein functioning as the principal receptor for glycosaminoglycan hyaluronate (HA) and playing an important role in cell-extracellular matrix interactions.

CD44 is a ubiquitous multistructural and multifunctional cell surface adhesion molecule involved in cell-cell and cell-matrix interactions. Twenty exons are involved in the genomic organization of this molecule. The first five and the last five exons are constant, whereas the 10 exons located between this region are subjected to alternative splicing, resulting in the generation of a variable region. Differential utilization of the 10 variable region exons generate multiple isoforms (at least 20 are known) of different molecular sizes (85-230 kDa). The smallest CD44 molecule (85-95 kDa), which lacks the entire variable region, is standard CD44 (CD44s). As it is expressed mainly on cells of lymphohematopoietic origin, CD44s is also known as hematopoietic CD44 (CD44H). CD44s is a single chain molecule composed of a distal extracellular domain (containing the ligand binding sites), a membrane proximal region, a transmembrane spanning domain, and a cytoplasmic tail. The molecular sequence (with the exception of the membrane proximal region) displays high interspecies homology. After immunological activation, T lymphocytes and other leukocytes transiently upregulate CD44 isoforms expressing variant exons (designated CD44v). A CD44 isoform containing the last 3 exon products of the variable region (CD44V8-10, also known as epithelial CD44 or CD44E), is preferentially expressed on epithelial cells. The longest CD44 isoform expressing in tandem eight exons of the variable region (CD44V3-10) was detected in keratinocytes.

Hyaluronic acid (HA), an important component of the extracellular matrix (ECM), is the principal, but by no means only ligand of CD44. Other CD44 ligands include the ECM components collagen, fibronectin, laminin, and chondroitin sulfate. Mucosal addressin, serglycin, osteopontin, and the class 2 invarient chain are additional, ECM-unrelated, ligands of the molecule. However, the polymorphic nature of CD44 suggests that this list is far from complete. Such hypothetical, novel CD44 ligands could be involved in tumor cell-tumor cell, tumor cell-normal cell, or tumor cell-matrix interactions, and their identification may allow the design of competitive or antagonistic reagents with potential antimetastatic effect.

In tumor cells, an overproduction of large CD44v proteins by enhanced transcription of alternatively spliced variant CD44v mRNA has been observed. Increased expression of CD44v proteins correlates with advanced stages of human breast, uterine, cervical and colon cancer, and has been found in human carcinoma lines from lung, breast and colon. In breast and colon cancer, expression of epitopes encoded by exons V5 or V6 on primary tumors is an independent prognostic factor for poor patient survival (Dall et al. (1995), Int. J. Cancer 66: 471-477; Rodriguez et al. (1995), Int. J. Cancer 64: 347-354; Liu (1994), Cancer Letters 76: 63-69).

Cells from the human Namalwa cell line (a Burkitt lymphoma derived lymphocyte) do not produce CD44 and are not metastatic. Upon transfection with CD44v (exon v6) cDNA, they gain the potential to colonize the lungs in an experimental metastasis protocol. Monoclonal antibodies specific for the v6 epitope, injected intravenously, prevent the outgrowth of CD44v6 transfectants in the draining lymph nodes and the formation of metastases in the lung and elsewhere (Herrlich et al. (1993), Immunology Today 14(8): 395-399).

The hyaluronate receptor CD44 has been shown to be involved in lymphocyte homing during normal leucocyte circulation and during leucocyte extravasation into sites of tissue inflammation (Welsh et al. (1995) J. Cellular Physiol. 164: 605-612). Activated CD4⁺ or CD8 lymphocytes as well as non-activated lymphocytes express CD44s (standard) while CD44v (especially v6) is expressed only by memory T-lymphocytes CD4- and CD8- (Tiberghien et al. (1994), Immunology 83(4): 552-561). When activated, memory T-lymphocytes express large quantities of CD44v protein on their surface and produce strikingly higher amounts of interferon-gama (IFN-γ) and interleukin-4 (IL-4). Increased concentrations of IFN-γ and IL-4 may contribute to the formation of various autoimmune syndromes since these cytokines may effect the production of certain classes of antibodies (autoimmune antibodies) by augmenting MHC class II expression (Budd et al. (1991), Eur. J. Immunol. 21(4): 1081-1084).

The role of the CD44 receptor in autoimmune disease and inflammation has been studied in several animal models using monoclonal antibodies (Mabs) which have been directed against CD44s or CD44v (especially the v6 variant). In collagen type II-induced arthritis in mice, injection of a monoclonal anti-CD44 antibody suppressed the autoimmune disease (Verdrengh et al. (1995), Scan. J. Immunol. 42 (3) : 353-358).

Similar results were obtained in rats with experimentally induced-glomerulonephritis, immunized with bovine glomerular basement membrane proteins (Nishikawa et al. (1993), Lab. Invest. 68(2): 146-153) as well as in mice which develop a syndrome similar to systemic lupus erythematosus in man. This strain of mice is characterized by the progressive accumulation of CD4-CD8- T-cells which express increased levels of CD44v on their surface. These T-cells exhibit a high level of spontaneous cytolytic activity and contain high levels of serine esterase as compared with CD4⁺CD8⁺T-cells. Treatment of these mice with anti-CD44v Mab augments the cytolytic activity of the CD4⁻CD8⁻ T-cells of the mice and causes suppression of their systemic lupus erythematosus like syndrome (Wang et al. (1993), Int. Immunol. 5(4): 361-369) .

### Methods

### Antibody library source

A human antibody synthetic library as described in Example 7 was used.

### Panning strategy and results

(A) Namalwa cells transfected with CD44V3-10 (Nam.+CD44V3-10) were panned with the single pot phagemid antibody library as follows: for the first round of selection, approximately 5 x 10⁶ Nam.+CD44V3-10 cells were washed, blocked with PBS containing 4% skim milk and M13 and panned with 10¹² phagemids from N1 library. After incubation at 4°C for 3h with slow agitation, cells were washed four times with PBS and the bound phages were eluted from the cells at room temperature for 10 min with 0.1M glycin pH-2.2. After neutralization, cells were spun, discarded, and the supernatant containing the eluted phages was collected and amplified by infecting E.coli bacteria (TG-1 strain) and plating them on ampicillin agar plates. Phagemids were rescued by superinfecting exponentially growing amp^{r} bacteria with helper phage (VSC-M13, Stratagene). This amplified stock was used for four additional rounds of panning on Namalwa cells as negatives and Nam.+CD44V3-10, with one amplification step after the second panning and without amplification step after the further rounds.
   4 peptides were isolated by this experiment:
   **Amino acid sequence displayed in the CDR3 of clone IG-NC-1:** 
Gly Thr Leu Asn Gln Cys Gly Arg Ile Asn
1 2 3 4 5 6 7 8 9 10
   **Amino acid sequence displayed in the CDR3 of clone IG-NC-3:** 
Cys Ala Val Glu Ala Ala Gly Pro Val Arg Val Leu
1 2 3 4 5 6 7 8 9 10 11 12
   **Amino acid sequence displayed in the CDR3 of clone IG-NC-14:** 
Ser Gly Ser Leu Gly Arg Ser Leu Glu
1 2 3 4 5 6 7 8 9
   **Amino acid sequence displayed in the CDR3 of clone IG-NC-11:** 
Thr Gly Asp Glu
1   2   3   4
(B) Namalwa/CHO-K1 cells which transfected with CD44V3-10 were panned with the single pot phagemid antibody library using the protocol of work described in (A) in the following approach:
   For the first round of selection approximately 5 x 10⁶ Nam.+CD44V3-10 cells were washed, blocked with PBS containing 4% skim milk and M13 and panned with 10¹² phagemids from N1 library. The bound phages were eluted from the cells and the supernatant containing the eluted phages was collected and divided into two steps: one including amplification step by infecting E.coli bacteria, and second step was done without amplification. For the second round of selection approximately 5 x 10⁶ Namalwa/CHO-K1 cells were washed, blocked with PBS containing 4% skim milk and M13 and panned with phagemids eluted from the first selection round. The phagemids which did not bound to the Namalwa/CHO-K1 cells were incubated with Namalwa/CHO-K1 cells transfected with CD44V3-10 respectively. The second round of selection was repeated twice.
   6 peptides were isolated by this experiment:
   **Amino acid sequence displayed in the CDR3 of clone IG-NC-39:** 
Phe Lys Ala Ser Arg His Ser
1   2   3   4   5   6   7
   **Amino acid sequence displayed in the CDR3 of clone IG-NC-467:** 
Ile His Met Arg Ala
1 2 3 4 5
   **Amino acid sequence displayed in the CDR3 of clone IG-NC-471:** 
Lys Asn Ala Asn
1 2 3 4
   **Amino acid sequence displayed in the CDR3 of clone IG-NC-650:** 
Met Arg Ala Pro Val Ile
1 2 3 4 5 6
   **Amino acid sequence displayed in the CDR3 of clone IG-NC-651:** 
Gly Ile Lys Gly Leu Asp Glu
1 2 3 4 5 6 7
   **Amino acid sequence displayed in the CDR3 of clone IG-NC-652:**

Cys Lys Trp Glu Lys Arg
1 2 3 4 5 6

All the peptides isolated are synthesized as described in Example 4. These peptides (or peptide mimetics thereof), alone or linked to an anti-cancer agent are used as anti-metastatic agents. In addition, these peptides are used for the treatment of autoimmune diseases, alone or linked to a suitable drug to form a drug-peptide conjugate.

### EXAMPLE 9: Isolation of peptides which specifically bind to undetermined targets on the blood vessels of Kaposi sarcoma (KS) tumor-bearing nude mice in vivo.

### Phage selection strategy:

For the induction of KS tumors we used a KS cell line which was isolated from a non-HIV-infected patient, and which was recently shown to carry several markers of HIV-infected KS cells (Herndier et al. (1994), Aids 8(5): 575-581). The KS cells (5x10⁶) as a mixture in Matri-gel (1:1 v/v) were injected into nude mice. After approximately 12-14 days, when well defined localized tumors were visualized (about 1.2 cm in diameter), a mixture of the 15-mer library (described in Example 1) together with M13 bacteriophage (10¹²:10¹³, respectively) was injected into the tail-vein. After 4 minutes, the animal vascular system was perfused with PBS for an additional 4 minutes and then sacrificed. Tumors were excised, weighed, and kept on ice for all subsequent manipulations. Following homogenization and extensive washing, phage elution was carried out with starved bacteria. The eluated phage was titrated, amplified, and then subjected to additional rounds of biopanning in KS tumor bearing mice.

### Results of biopanning in vivo

The KS-tumor eluted phages from two independent experiments (designated MKS1 and MKS2), following three rounds of biopanning *in vivo*, were analyzed by DNA sequencing. The sequencing results indicated that almost 100% of the clones at round 3 in both experiments, were identical to clone TUV-R4B*#1 (Example 1), which was also selected on umbilical vein and artery, *ex vivo*.

### Organ distribution of selected phages:

Mixtures of selected phage and M13 bacteriophage (5x10¹⁰ and 5x10¹¹, respectively) were injected into the tail vein of KS-tumor bearing mice, and after one round of panning, tumor and brain tissue were excised, washed, and treated for phage elution and titration as described above. The results of three parallel experiments, with various phage mixtures, are demonstrated in Figure 1.
These results demonstrate that the tumor-distribution of clone TUV-R4B*#1 is 4-5 fold higher than the brain-distribution. No such results were obtained with the two other clones, which demonstrate similar low level of binding to both tumor and brain tissues.

### EXAMPLE 10: Isolation of peptides which specifically bind to undetermined targets on Kaposi sarcoma cells in culture.

Biopanning was conducted on a Kaposi sarcoma cell line (Herndier et al. (1994), Aids 8(5): 575-581) according to protocol EC-1 (section 1.5.5 in Example 1) with the following modifications: the selection was carried out with a mixture of 2x10¹⁰ phages of the 15-mer library (Example 1) and 5x10¹¹ M13 wild-type phage. Phage selection (incubation) was conducted at room-temperature for 60 minutes. Phage elution from the cells was carried out with starved bacteria only. Amplified phage was subjected to two additional cycles of biopanning. The DNA of 18 single phage clones from the eluate of the third cycle of biopanning was subjected to DNA sequencing analysis.
10/28 clones were unique while 8/28 clones were identical and were designated TSKC-R3#3.
**Amino acid sequence displayed by clone TSKC-R3#3:**

Ala Arg Leu Ser Pro Thr Met Val His Pro Asn Gly Ala Gln Pro
1               5                   10                  15

### EXAMPLE 11: Isolation of peptides that specifically bind to a predetermined target (fibronectin)

Biopanning was conducted on human plasmatic fibronectin (FN), (see eg. Akiyama and Yamada (1987), Adv. Enzymol 57: 1-57) essentially according to protocol EC-1 (section 1.5.5 in Example 1) with the following modifications: 35mm plates were coated overnight at 4°C with 25µg FN solution (in 5ml PBS). Plates were blocked with 5% Human Serum (HS) in PBS for 2 hours at room temperature, and washed 3 times with washing solution (5% HS-0.2% Tween in PBS). The phage selection was carried out with a mixture of 2x10¹⁰ TTU of the 15-mer library (Example 1) and 2x10¹¹ PFU M13. Phage selection (incubation) was conducted at room-temperature for 60 minutes. Plates were washed 5 times with washing solution (5% HS-0.2% Tween in PBS). Phage elution was carried out in two steps for 10 minutes each at room temperature with:
Elution buffer 1: 0.2N glycine, pH2.2-0.5% Tween in PBS, followed by immediate neutralization of the acidic eluate with Trisma base.
Elution buffer 2: 0.05% EDTA in PBS.
Elutions 1 and 2 were mixed before bacterial infection and amplification.

The DNA of 16 single phage clones isolated from the third cycle of biopanning was analyzed by DNA sequencing.
8/16 clones were identical and were designated TFN-R3-#150.
4/16 clones were identical and were designated TFN-R3-#111.
4/4 clones were unique.
**Amino acid sequence displayed by clone TFN-R3-#150:** 
Ala Leu Gly Gly Phe Arg Pro Phe Trp Ser Tyr Gly Gly Leu Ser
1               5                   10                  15
**Amino acid sequence displayed by clone TFN-R3-#111:**

Met Gly Ala Asp Asp Ala Pro His Tyr Trp His Pro Val Trp Thr
1 2 3 4 5 6 7 8 9 10 11 12 13 14 15

### EXAMPLE 12: Isolation of peptides which specifically bind to undetermined targets on human Acute Myeloid Leukemia (AML) cells.

### Biopanning protocol

Frozen AML lymphocytes, which were originally isolated by Ficol-density centrifugation from the peripheral blood cells of an Acute Myeloid Leukemic patient, were quickly thawed at 37°C water bath, and washed in washing medium (DMEM-10% Human serum (HS)). The viability estimated by trypan blue exclusion was 75-90%. The AML cells were resuspended in selection medium (Hanks-2 mM HEPES, pH 6.9 and 2% HS). Approximately, 5×10¹¹ TTU (from the 15 or the 6-mer library, Example 1) were added to 5X10⁶ cells in a final volume of 1.4ml medium in an Eppendorf tube, and the mixture was incubated at 4°C, for 60 minutes while rocking. The cells were then washed with DMEM medium, and the Eppendorf tube was changed after the second washing. Washing was repeated four times. Phage elution from the cells was carried out with Trypsin-EDTA (200µl, Gibco) at 37°C for 15 minutes, followed by the addition of HS (10% final concentration) for trypsin neutralization. Phage titrations in the input and the eluate (output), and phage amplification for additional rounds of biopanning, were carried out according to G. Smith (Virology 167: 156-165, 1988).

### Biopanning on AML lymphocytes with the 15-mer library:

In rounds 2-4, the amplified output (eluted phage) was used as the input phage for the subsequent round of biopanning.

The DNA of 28 single clones was analyzed by DNA sequencing after the fifth round of selection.
17/28 clones were identical and were designated EAML.5.73. 11/28 clones were unique.
**Amino acid sequence displayed by clone EAML.5.73.:**

Cys Thr Arg Leu Gly Ala Ala Ala Gly Arg Cys Asp Val Gly Leu
1 2 3 4 5 6 7 8 9 10 11 12 13 14 15

The DNA of 9 clones was analyzed by DNA sequencing after the sixth round of selection.
2/9 clones were identical and were designated EAML.6.1.
**Amino acid sequence displayed by clone EAML.6.1.:**

Ser Asn Leu Asn Arg Phe Val Phe Ala Phe Trp Asp Gly Pro Ala
1 2 3 4 5 6 7 8 9 10 11 12 13 14 15

### Biopanning on AML lymphocytes with a 1:1 mixture of the 15 and 6-mer libraries

28 single colonies from round 6 were sequenced.
20/28 clones were identical and were designated EAML.6.108.
**Amino acid sequence displayed by clone EAML. 6.108:**

Arg Leu Phe Met Leu Gly
1   2   3   4   5   6

An additional experiment was carried out as above, in which 5 single colonies from round 6 were sequenced.
3/5 clones were identical and were designated EAML 6.2.
2/5 clones were identical and were designated EAML 6.4
**Amino acid sequence displayed by clone EAML.6.2.:** 
Leu His Gly Phe Ala Ser His Lys Asp Gly Pro Leu Ile Pro Ala
1 2 3 4 5 6 7 8 9 10 11 12 13 14 15
**Amino acid sequence displayed by clone EAML.6.4.:**

Leu Val Phe Val Lys Asn His Pro Leu Val Pro Phe Gly Ser Pro
1 2 3 4 5 6 7 8 9 10 11 12 13 14 15

The comparative binding of the AML-selected clones EAML.5.73 and EAML.6.108 to AML cells from three different AML patients was 10-100 times higher than the original 15-mer and 6-mer libraries.

### EXAMPLE 13: Isolation of peptides which specifically bind to undetermined targets on human chronic lymphoid leukemia (T-CLL) cells.

### Biopanning strategy:

Biopanning on CLL3 cells was conducted with the 15-mer library (Example 1, 2x10⁶ cells, 5x10¹⁰ phage, 2x10¹² M13, 4°c, 60min, Hanks-Heppes). Biopanning followed by amplification was carried out for several rounds.
Round 1: Elution of bound phage with acid-glycine followed by trypsin-EDTA. The two eluates were mixed, amplified and then used for the second round.
Round 2: Phage eluted with acid-glycine and then with trypsin-EDTA. Each eluate was amplified and used separately for the next rounds of biopanning.
Round 3-5: When the amplified acid eluate from round 2 was used for biopanning, phage elution was carried with acid-glycine. When the amplified trypsin-EDTA eluate was used for biopanning, bound phage was washed with acid-glycine, and elution was carried out with trypsin-EDTA.

Specific phage enrichment was monitored by DNA sequencing and by binding assays.

Three peptides were isolated by acid elutions:
**Amino acid sequence displayed by clone TCLL.HA4.6:** 
Pro Ile Trp His Gly Asp Ser Gly Val Tyr Ser Ser Phe Phe Pro
1 2 3 4 5 6 7 8 9 10 11 12 13 14 15
**Amino acid sequence displayed by clone TCLL.HA5.5:** 
Arg Asn Thr Leu Pro His Phe Ser Phe Gly Pro Arg Leu Tyr Arg
1               5                   10                  15
**Amino acid sequence displayed by clone TCLL.HA3.11:**

Gly Leu Ser Asp Gly pro Tyr Tyr Ser Phe Ser Leu Phe Arg Phe
1 2 3 4 5 6 7 8 9 10 11 12 13 14 15

Two peptides were isolated by trypsin-EDTA elutions:
**Amino acid sequence displayed by clone TCLL.HT3.2:** 
Gly Gly Ala Ala Gly Gly Tyr Leu Arg Val Phe Ala Gly Val Arg
1 2 3 4 5 6 7 8 9 10 11 12 13 14 15
**Amino acid sequence displayed by clone TCLL.HT4.13:**

Gly Tyr Asp Cys Trp Asp Cys Pro Phe Ser Phe Arg Gly Ser Val
1 2 3 4 5 6 7 8 9 10 11 12 13 14 15

### EXAMPLE 14: Isolation of peptides that specifically bind to Thrombospondin

### Background

Thrombospondin (TSP) is a glycoprotein which is stored in platelets α-granules, and generally released in response to thrombin stimulation. TSP functions as a cell adhesion molecule as well as a modulator of cell movement and proliferation. There is growing evidence that TSP functions as a natural modulator of angiogenesis, leading to positive or negative regulation of angiogenesis, and as a function of context and TSP concentration. Moreover, TSP is overexpressed by tumors, and more specifically, high levels of TSP were observed in association with malignant carcinomas of the breast. TSP molecules are also a transient component of extracellular matrix in developing and repairing tissues. This process is mediated by binding of the amino-terminal heparin-binding domain of TSP to the extracellular matrix and cell surface proteoglycans.

### Biopanning strategy

Biopanning was conducted on the recombinant heparin-binding-domain of human TSP-1 (18 kD) designated rTSP-HBD1 (Vogel et al. (1994), J.Cell.Biochem., 53:75), which spans amino acids 1-174 of human TSP-1. Biopanning was carried out essentially according to protocol EC-1 (Example 1) with the following modifications: For round 1, 35mm plates were coated overnight at. 4°C with 25µg solution (in 5ml PBS) of rTSP-HBD1. Plates were blocked with 2% skim-milk (SM) in PBS for 45 minutes at 37°C and 45 minutes at room temperature, and washed 3 times with washing solution (PBS). Phage selection was carried out with a mixture of approximately 2x10¹⁰ TTU of the 15-mer library (Example 1) and 2x10¹¹ PFU M13, or with 10¹¹ Nissim N-1 library (Example 7) in 2% SM-PBS. Incubation was carried out at room temperature for 90 minutes. Plates were washed 5 times with 0.2% Tween-PBS, followed by 5 washings with PBS. Phage elution was carried out for 10 min at room temperature with:
Elution buffer 1: 0.2N glycine (pH2.2)-0.2% Tween, followed by immediate neutralization with Trisma base.
Elution buffer 2: 5x10⁹ starved K91 bacteria for 30 min at room temperature.

Elutions 1 and 2 were mixed before amplification. For the subsequent rounds, the protocol was as described above except that the plates were coated with 5*µ*g solution of rTSP-HBD1, and phage incubation was carried out for 45 minutes. Elutions 1 and 2 were amplified and used separately for rounds 3 and onwards.

DNA analysis of 17 phage clones (Nissim N-1 library) was carried out after round 3 (elution buffer 2):
3/17 clones were identical and were designated TTSP.R3N.B5.
**Amino acid sequence displayed in the CDR3 of TTSP.R3N.B5:** 
Ser Lys Arg Ala Asn Gly Phe Arg Gly Val Ser
1 2 3 4 5 6 7 8 9 10 11
1/17 clones was designated TTSP.R3N.B3.
**Amino acid sequence displayed in the CDR3 of TTSP.R3N.B3:** 
Ile Lys His Tyr Gly Arg Lys Arg Asn
1 2 3 4 5 6 7 8 9
1/17 clones was designated TTSP.R3N.B4.
**Amino acid sequence displayed in the CDR3 of TTSP.R3N.B4:**

Val Lys Lys Phe Lys Gly Gly Gln Arg Val

1 2 3 4 5 6 7 8 9 10

| clone | Frequency | Sequence |
|---|---|---|
| TTSP.R3N.B5 | 3/17 | S K R A N G F R G V S |
| TTSP.R3N.B3 | 1/17 | I K H Y G R K R N |
| TTSP.R3N.B4 | 1/17 | V K K F K G G Q R V |

The bold and italics signs designate the consensus amino acids of the selected peptide ligands.

## Claims

1. A non-naturally occurring pharmaceutically active peptide for targeting a pharmaceutical agent or imaging agent to a tissue or organ comprising the amino acid sequence Met Arg Ala Pro Val Ile.

2. The peptide of claim 1 having a pharmaceutical agent linked thereto.

3. The peptide according to claim 2 wherein the pharmaceutical agent is a polypeptide and is linked to the peptide by a peptide linkage.

4. The peptide according to claim 2 or 3, wherein the pharmaceutical agent is a toxin, an anti-cancer agent, an antiangiogenic compound, a cardiovascular agent, an agent used in a neurological disorder, a liver disease agent, a kidney disease agent or a radioisotope.

5. The peptide according to anyone of claims 2 to 4 wherein the pharmaceutical agent is a recombinant protein.

6. The peptide of any one of claims 1 to 5 labeled with an imageable marker.

7. The peptide according to claim 6 wherein the imageable marker comprises a radioactive isotope, an element which is opaque to X-rays or a paramagnetic ion.

8. The peptide of claim 7 wherein the radioactive isotope comprises indium-111, technetium-99, iodine-123, iodine-125, iodine-131, krypton-81m, xenon-33 or gallium-67.

9. A composition comprising a peptide according to any one of claims 1 to 8 and optionally a pharmaceutically acceptable carrier.

10. The composition according to claim 9 wherein the pharmaceutical agent of claim 2 is a recombinant protein.

11. A method for the identification of a peptide of claim 1 which comprises:
(a) incubating a phage display peptide library with an isolated organ;
(b) washing the isolated organ to remove unbound phages;
(c) eluting bound phage from the isolated organ;
(d) amplifying the resulting bound phage; and
(e) determining the displayed peptide sequence of the bound phage so as to identify the peptide.

12. The method according to claim 11, wherein the phage display peptide library is a 15-mer library, or a 6-mer library.

13. The method according to claims 11 or 12 wherein the elution medium is a compound selected from acid, urea, Octyl, trypsin or tween.

14. The method according to any one of claims 11 to 13 wherein the isolated organ is a perfused organ.

15. The method according to any one of claims 11 to 14 wherein the isolated organ is an artery, a vein, placenta, tumor tissue, kidney, heart, liver, or central nervous system

16. Use of the peptide of any one of claims 6 to 8 for the preparation of a composition for imaging an organ.

17. Use of the peptide of any one of claims 1 to 5 for the production of a pharmaceutical composition for treating an organ.

18. The use of the peptide according to claim 17 wherein the pharmaceutical composition is adapted for contacting the organ to be treated with an effective amount of the peptide under conditions such that the peptide binds to the organ and thereby treating the organ.

19. The use of the peptide according to claims 16 to 18 wherein the isolated organ is an artery, a vein, placenta, tumor tissue, kidney, heart, liver, or central nervous system.

20. An ex vivo method for imaging an organ which comprises:
(i) contacting the organ to be imaged with the peptide of any one of claims 6 to 8 under conditions such that the peptide binds to the organ;
(ii) imaging the bound peptide; and
(iii) thereby imaging the organ.

21. The method of claim 20 wherein the organ is an artery, a vein, placenta, tumor tissue, kidney, heart or liver.

22. The use of the peptide according to claims 18 or 19 or the method of claim 20 or 21 wherein the artery is a umbilical cord artery, a radial artery, a coronary artery or a mammary artery; or wherein the vein is an umbilical cord vein, a safenal vein or a femoral vein.

23. The use or the method of claim 22 wherein the artery is a damaged artery.

24. The use or the method of claim 23 wherein the damaged artery is a damaged coronary artery.

25. A method of synthesizing the peptide of claim 1 which comprises joining the amino acids of the peptide in the proper order.

26. A method of claim 25 which comprises:
(a) identifying the peptide by the steps of the method of claim 11; and
(b) synthesizing the peptide by the steps of the method of claim 25.

27. Use of an effective amount of the peptide of any of claims 1 to 8 in the manufacture of a medicament for targeting a pharmaceutical agent or an imaging agent to an organ or tissue.

28. A pharmaceutical composition comprising an amount of the peptide of any of claims 1 to 8 effective to target a pharmaceutical agent or an imaging agent to an organ or tissue.

## Patentansprüche

1. Nicht-natürlich vorkommendes pharmazeutisch aktives Peptid zur Zielrichtung eines pharmazeutischen Wirkstoffs oder bildgebenden Mittels auf ein Gewebe oder Organ, umfassend die Aminosäuresequenz Met Arg Ala Pro Val Ile.

2. Peptid nach Anspruch 1, das einen pharmazeutischen Wirkstoff daran gebunden hat.

3. Peptid nach Anspruch 2, wobei der pharmazeutische Wirkstoff ein Polypeptid ist und über eine Peptidbindung an das Peptid gebunden ist.

4. Peptid nach Anspruch 2 oder 3, wobei der pharmazeutische Wirkstoff ein Toxin, ein Antikrebsmittel, eine anti-angiogenetische Verbindung, ein kardiovaskulärer Wirkstoff, ein Wirkstoff, der bei einer neurologischen Erkrankung verwendet wird, ein Wirkstoff gegen eine Erkrankung der Leber, ein Wirkstoff gegen eine Erkrankung der Niere oder ein Radioisotop ist.

5. Peptid nach einem der Ansprüche 2 bis 4, wobei der pharmazeutische Wirkstoff ein rekombinantes Protein ist.

6. Peptid nach einem der Ansprüche 1 bis 5, das mit einem bildgebenden Marker markiert ist.

7. Peptid nach Anspruch 6, wobei der bildgebende Marker ein radioaktives Isotop, ein Element, das für Röntgenstrahlung undurchlässig ist, oder ein paramagnetisches Ion umfasst.

8. Peptid nach Anspruch 7, wobei das radioaktive Isotop Indium-111, Technetium-99, Jod-123, Jod-125, Jod-131, Krypton-81m, Xenon-33 oder Gallium-67 umfasst.

9. Zusammensetzung, umfassend ein Peptid nach einem der Ansprüche 1 bis 8 und gegebenenfalls einen pharmazeutisch verträglichen Träger.

10. Zusammensetzung nach Anspruch 9, wobei der pharmazeutische Wirkstoff nach Anspruch 2 ein rekombinantes Protein ist.

11. Verfahren zur Identifizierung eines Peptides nach Anspruch 1 umfassend:
(a) Inkubieren einer Phagen-Display-Peptidbibliothek mit einem isolierten Organ;
(b) Waschen des isolierten Organs, um ungebundene Phagen zu entfernen;
(c) Eluieren des gebundenen Phagen aus dem isolierten Organ;
(d) Amplifizieren der erhaltenen gebundenen Phagen; und
(e) Bestimmen der präsentierten Peptidsequenz des gebundenen Phagens zum Identifizieren des Peptids.

12. Verfahren nach Anspruch 11, wobei die Phagen-Display-Peptidbibliothek eine 15-Mer-Bibliothek oder eine 6-Mer-Bibliothek ist.

13. Verfahren nach Anspruch 11 oder 12, wobei das Elutionsmedium eine Verbindung ausgewählt aus Säure, Harnstoff, Octyl, Trypsin oder Tween ist.

14. Verfahren nach einem der Ansprüche 11 bis 13, wobei das isolierte Organ ein perfundiertes Organ ist.

15. Verfahren nach einem der Ansprüche 11 bis 14, wobei das isolierte Organ eine Arterie, eine Vene, Plazenta, Tumorgewebe, Niere, Herz, Leber oder Zentralnervensystem ist.

16. Verwendung des Peptids nach einem der Ansprüche 6 bis 8 für die Herstellung einer Zusammensetzung zur Bildgebung eines Organs.

17. Verwendung des Peptides nach einem der Ansprüche 1 bis 5 für die Herstellung eines Arzneimittels zur Behandlung eines Organs.

18. Verwendung des Peptids nach Anspruch 17, wobei das Arzneimittel angepasst ist für das Inkontaktbringen des zu behandelnden Organs mit einer wirksamen Menge des Peptids unter Bedingungen, so dass das Peptid an das Organ bindet, und **dadurch** das Behandeln des Organs.

19. Verwendung des Peptids nach den Ansprüchen 16 bis 18, wobei das isolierte Organ eine Arterie, eine Vene, Plazenta, Tumorgewebe, Niere, Herz, Leber oder Zentralnervensystem ist.

20. Ex-vivo-Verfahren zur Bildgebung eines Organs, umfassend:
(i) Inkontaktbringen des darzustellenden Organs mit einem Peptid nach einem der Ansprüche 6 bis 8 unter Bedingungen, so dass das Peptid an das Organ bindet;
(ii) Bildgebung des gebundenen Peptids, und
(iii) **dadurch** Bildgebung des Organs.

21. Verfahren nach Anspruch 20, wobei das Organ eine Arterie, eine Vene, Plazenta, Tumorgewebe, Niere, Herz oder Leber ist.

22. Verwendung des Peptids nach Anspruch 18 oder 19 oder des Verfahrens nach Anspruch 20 oder 21, wobei die Arterie eine Nabelschnurarterie, eine radiäre Arterie, eine koronare Arterie oder eine Brustarterie ist, oder wobei die Vene eine Nabelschnurvene, eine saphenale Vene oder eine femorale Vene ist.

23. Verwendung oder Verfahren nach Anspruch 22, wobei die Arterie eine verletzte Arterie ist.

24. Verwendung oder Verfahren nach Anspruch 23, wobei die verletzte Arterie eine verletzte koronare Arterie ist.

25. Verfahren zum Synthetisieren des Peptids nach Anspruch 1, umfassend das Verknüpfen der Aminosäuren zu einem Peptid in der richtigen Reihenfolge.

26. Verfahren nach Anspruch 25, umfassend:
(a) Identifizieren des Peptids durch die Schritte des Verfahrens nach Anspruch 11; und
(b) Synthetisieren des Peptids durch die Schritte des Verfahrens nach Anspruch 25.

27. Verwendung einer wirksamen Menge des Peptids nach einem der Ansprüche 1 bis 8 für die Herstellung eines Arzneimittels zur Zielrichtung eines pharmazeutischen Wirkstoffs oder bildgebenden Mittels auf ein Organ oder Gewebe.

28. Arzneimittel, umfassend eine Menge des Peptids nach einem der Ansprüche 1 bis 8, die wirksam ist, um einen pharmazeutischen Wirkstoff oder ein bildgebendes Mittel auf ein Organ oder Gewebe zielzurichten.

## Revendications

1. Peptide pharmaceutiquement actif non naturel pour cibler un agent pharmaceutique ou agent d'imagerie sur un tissu ou un organe comprenant la séquence d'acides aminés Met Arg Ala Pro Val Ile.

2. Peptide de la revendication 1 ayant un agent pharmaceutique lié à lui-même.

3. Peptide selon la revendication 2 dans lequel l'agent pharmaceutique est un polypeptide et est lié au peptide par une liaison peptidique.

4. Peptide selon les revendications 2 ou 3 dans lequel l'agent pharmaceutique est une toxine, un agent anticancéreux, un composé antiangiogénique, un agent cardiovasculaire, un agent utilisé dans les troubles neurologiques, un agent contre une maladie hépatique, un agent contre une affection rénale ou un radio-isotope.

5. Peptide selon l'une quelconque des revendications 2 à 4 dans lequel l'agent pharmaceutique est une protéine recombinée.

6. Peptide selon l'une quelconque des revendications 1 à 5 marqué avec un marqueur décelable par imagerie.

7. Peptide selon la revendication 6 dans lequel le marqueur décelable par imagerie comprend un isotope radioactif, un élément qui est opaque aux rayons X ou un ion paramagnétique.

8. Peptide de la revendication 7 dans lequel l'isotope radioactif comprend l'indium 111, le technétium 99, l'iode 123, l'iode 125, l'iode 131, le krypton 81 m, le xénon 33 ou le gallium 67.

9. Composition comprenant un peptide selon l'une quelconque des revendications 1 à 8 et facultativement un vecteur pharmaceutiquement acceptable.

10. Composition selon la revendication 9 dans laquelle l'agent pharmaceutique de la revendication 2 est une protéine recombinée.

11. Procédé pour l'identification d'un peptide de la revendication 1 qui comprend:
(a) l'incubation d'une banque de peptides exposés à la surface de phages avec un organe isolé ;
(b) le lavage de l'organe isolé afin d'éliminer les phages non liés ;
(c) l'élution des phages liés de l'organe isolé ;
(d) l'amplification des phages liés résultants ; et
(e) la détermination de la séquence peptidique exposée à la surface des phages liés afin d'identifier le peptide.

12. Procédé selon la revendication 11 dans lequel la banque de peptides exposés à la surface de phages est une banque de pentadécamères ou une banque d'hexamères.

13. Procédé selon les revendications 11 ou 12 dans lequel le milieu d'élution est un composé choisi parmi un acide, l'urée, l'Octyl, la trypsine ou le Tween.

14. Procédé selon l'une quelconque des revendications 11 à 13 dans lequel l'organe isolé est un organe perfusé.

15. Procédé selon l'une quelconque des revendications 11 à 14 dans lequel l'organe isolé est une artère, une veine, le placenta, un tissu tumoral, le rein, le coeur, le foie ou le système nerveux central.

16. Utilisation du peptide de l'une quelconque des revendications 6 à 8 pour la préparation d'une composition pour observer un organe par imagerie.

17. Utilisation du peptide de l'une quelconque des revendications 1 à 5 pour la production d'une composition pharmaceutique pour le traitement d'un organe.

18. Utilisation du peptide selon la revendication 17 dans laquelle la composition pharmaceutique est adaptée pour sa mise au contact de l'organe à traiter avec une quantité efficace du peptide, dans des conditions telles que le peptide se fixe à l'organe et de façon à traiter l'organe.

19. Utilisation du peptide selon les revendications 16 à 18 dans laquelle l'organe isolé est une artère, une veine, le placenta, un tissu tumoral, le rein, le coeur, le foie ou le système nerveux central.

20. Procédé *ex vivo* pour observer un organe par imagerie qui comprend :
(i) la mise en contact de l'organe à observer par imagerie avec le peptide de l'une quelconque des revendications 6 à 8 dans des conditions telles que le peptide se lie à l'organe ;
(ii) l'observation, par imagerie du peptide lié ; et
(iii) de ce fait l'observation par imagerie de l'organe.

21. Procédé de la revendication 20 dans lequel l'organe est une artère, une veine, le placenta, un tissu tumoral, le rein, le coeur ou le foie.

22. Utilisation du peptide selon les revendications 18 ou 19 ou le procédé des revendications 20 ou 21 dans laquelle l'artère est une artère du cordon ombilical, une artère radiale, une artère coronaire ou une artère mammaire ; ou dans laquelle la veine et une veine du cordon ombilical, une veine saphène ou une veine fémorale.

23. Utilisation ou procédé de la revendication 22 dans lesquels l'artère est une artère lésée.

24. Utilisation ou procédé de la revendication 23 dans lesquels l'artère lésée est une artère coronaire lésée.

25. Procédé de synthèse du peptide de la revendication 1 qui comprend la liaison des acides aminés du peptide dans l'ordre approprié.

26. Procédé de la revendication 25 qui comprend :
(a) l'identification du peptide par les étapes du procédé de la revendication 11 ; et
(b) la synthèse du peptide par les étapes du procédé de la revendication 25.

27. Utilisation d'une quantité efficace du peptide de l'une quelconque des revendications 1 à 8 dans la fabrication d'un médicament pour cibler un agent pharmaceutique ou un agent d'imagerie sur un organe ou un tissu.

28. Composition pharmaceutique comprenant une quantité du peptide de l'une quelconque des revendications 1 à 8, efficace pour cibler un agent pharmaceutique ou un agent d'imagerie sur un organe ou un tissu.
